# EUROPEAN PATENT APPLICATION

(11) **EP 4 847 606 A1**
(43) Date of publication of application: **02.12.2026**
(21) Application number: 25745414.0
(22) Date of filing: 24.01.2025
(51) Int. Cl.: C12N 5/0789, A61K 35/28, A61P 7/00, A61P 35/00, A61P 37/04

(54) **COMPOSITION, CULTURE MEDIUM, CELL POPULATION, CELL PRODUCTION METHOD, AND THERAPEUTIC DRUG**

(30) Priority: 25.01.2024 JP 2024009804
(71) Applicant: Celaid Therapeutics Inc., Tokyo 113-8485 (JP); The University of Osaka, Suita-shi, Osaka 565-0871 (JP)
(72) Inventor: WATANABE Motoo, Tokyo 113-8485 (JP); ARAKAWA Nobuyuki, Tokyo 113-8485 (JP); NAKAYAMA Akiko, Tokyo 113-8485 (JP); OTSU Naoki, Tokyo 113-8485 (JP); ISHIHARA Haruna, Tokyo 113-8485 (JP); SUZUKI Takayoshi, Suita-shi, Osaka 565-0871 (JP); ITOH Yukihiro, Suita-shi, Osaka 565-0871 (JP); OKUDA Maho, Tokyo 113-8485 (JP)
(74) Representative: Germain Maureau
(86) International application number: PCT/JP2025/002267
(87) International publication number: WO 2025/159194

(57) **Abstract**

A composition (1) comprising component (A), which is a KDM5 degrader, and component (B), which is an LSD1 inhibitor, or a composition (2) comprising component (A), which is a KDM5 degrader, wherein, in composition (2), said component (A) comprises at least one component selected from the group consisting of a compound represented by the following general formula (I) and a salt thereof, and a compound represented by the following general formula (II) and a salt thereof.

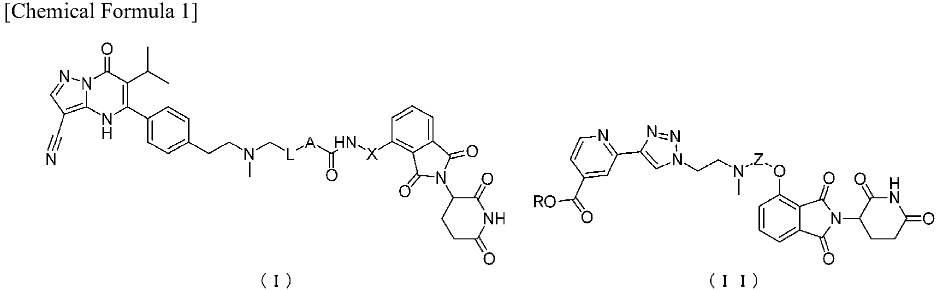

## Description

### TECHNICAL FIELD

The technical field of the present disclosure relates to a composition used for cell culture, a medium, a cell population, a method for producing cells, cells, and a therapeutic agent.

Priority is claimed on Japanese Patent Application No. 2024-009804, filed on January 25, 2024, the content of which is incorporated herein by reference.

The contents described in WO2024/210126, filed on April 2, 2024, and published on October 10, 2024, are incorporated herein by reference.

The contents described in "Aryl hydrocarbon receptor-dependent enrichment of a megakaryocytic precursor with a high potential to produce proplatelets," Catherine Strassel et al., Blood (2016) 127 (18):2231-2240, published on May 5, 2016, are incorporated herein by reference.

### BACKGROUND ART

Hematopoietic stem cells are stem cells capable of differentiating into blood lineage cells. A therapeutic method for diseases such as cancer and immunodeficiency is known, which involves transplanting hematopoietic stem cells into patients with said diseases.

Umbilical cord blood (CB) is known as a source of hematopoietic stem cells. For example, Patent Document 1 describes a method for obtaining hematopoietic stem cells from human umbilical cord blood (hereinafter, also referred to as "human CB"). Specifically, it describes selecting and culturing a population of EPCR⁺ cells from a starting population of human CB CD34⁺ cells. EPCR is a hematopoietic stem cell marker also known as CD201.

Furthermore, methods for culturing hematopoietic stem cells have been studied. Generally, in the culture of hematopoietic stem cells, an albumin-containing medium has been used to induce cell division. However, albumin has problems such as inhibition of stable maintenance undifferentiation and biological contamination. Therefore, research has been conducted on albumin-free culture methods. For example, Patent Document 2 describes the culture of human hematopoietic stem cells by adding PVA or Soluplus (registered trademark) instead of albumin. Patent Document 3 describes a method for producing megakaryocytes (cells that become platelets) from hematopoietic progenitor cells, characterized by culturing the hematopoietic progenitor cells in a medium containing a pyrimidoindole derivative.

### Citation List

### Patent Document

Patent Document 1: WO2017/205977
Patent Document 2: WO2021/149799
Patent Document 3: WO2024/210126

### SUMMARY OF INVENTION

### Technical Problem

Conventional methods were not sufficient for efficiently obtaining human hematopoietic stem cells. Furthermore, there have been few reports on albumin-free culture methods for human hematopoietic stem cells, and the available media were limited.

### Solution to Problem

In the course of their research, the present disclosers studied methods for culturing hematopoietic stem cells. As a result, they were able to successfully proliferate hematopoietic stem cells by culturing human CB CD34⁺ cells in an albumin-free medium with a specific composition different from conventional ones. Furthermore, the obtained cultured fraction was a cultured fraction enriched with CD201⁺ or CD90⁺ (both are hematopoietic stem cell markers) hematopoietic stem cells. The present disclosers completed the present disclosure based on this finding.

The present disclosure has the following embodiments.
[1] A composition (1) comprising component (A), which is a KDM5 degrader, and component (B), which is an LSD1 inhibitor, or
   a composition (2) comprising component (A), which is a KDM5 degrader, wherein
   in composition (2), said component (A) comprises at least one component selected from the group consisting of a compound represented by the following general formula (I) and a salt thereof, and a compound represented by the following general formula (II) and a salt thereof: 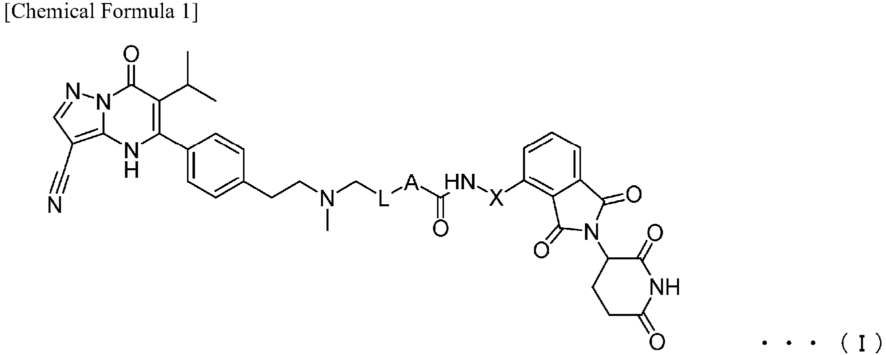
   in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)ₛ-, - (CH₂CH₂CH₂O)ₛ-, -(CH(CH₃)CH₂O)ₛ-, or -(CH₂)ₜO-, s is a number from 1 to 8, and t is a number from 2 to 10; 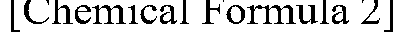 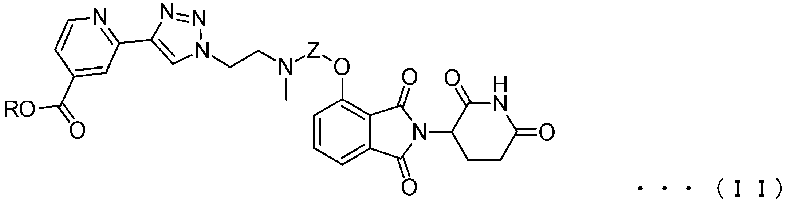
   in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.
[2] The composition according to [1], wherein said composition is composition (1), and
   said component (A) comprises at least one component selected from the group consisting of a compound represented by the following general formula (I) and a salt thereof, and a compound represented by the following general formula (II) and a salt thereof: 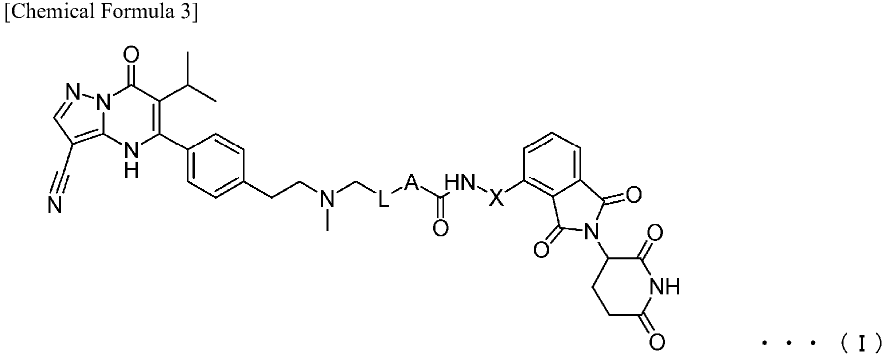
   in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10; 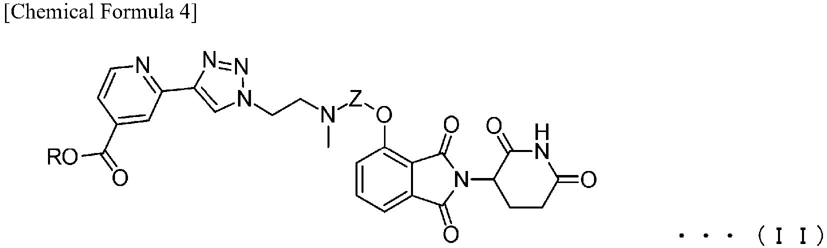
   in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.
[3] The composition according to [1], wherein
   in said general formula (I), A is a single bond or phenylene group, L is a linear alkylene group having 3 to 10 carbon atoms, X is a group represented by -(CH₂CH₂O)□-, -(CH₂CH₂CH₂O)□-, -(CH(CH₃)CH₂O)□-, or -(CH₂)ₜO-, s is a number from 1 to 5, and t is a number from 2 to 10, and
   in said general formula (II), R is a hydrogen atom, or a linear alkyl group having 1 to 6 carbon atoms, and Z is a substituted or unsubstituted linear alkylene group having 2 to 18 carbon atoms, or a substituted or unsubstituted branched alkylene group having 2 to 18 carbon atoms.
[4] The composition according to [1], wherein
   in said general formula (I), A is a single bond, p-phenylene group, or m-phenylene group, L is a linear alkylene group having 3 to 10 carbon atoms, X is a group represented by -(CH₂CH₂O)□-, -(CH₂CH₂CH₂O)□-, or - (CH₂)ₜO-, s is a number from 1 to 5, and t is a number from 2 to 10, and
   in said general formula (II), R is a linear alkyl group having 1 to 6 carbon atoms, and Z is a substituted or unsubstituted linear alkylene group having 3 to 16 carbon atoms, or a substituted or unsubstituted branched alkylene group having 3 to 16 carbon atoms.
[5] The composition according to [1], wherein said component (A) is at least one selected from the group consisting of a compound represented by the following general formula (I-1) and a salt thereof, a compound represented by the following general formula (I-2) and a salt thereof, a compound represented by the following general formula (I-3) and a salt thereof, and a compound represented by the following general formula (II-1) and a salt thereof: 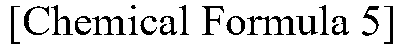 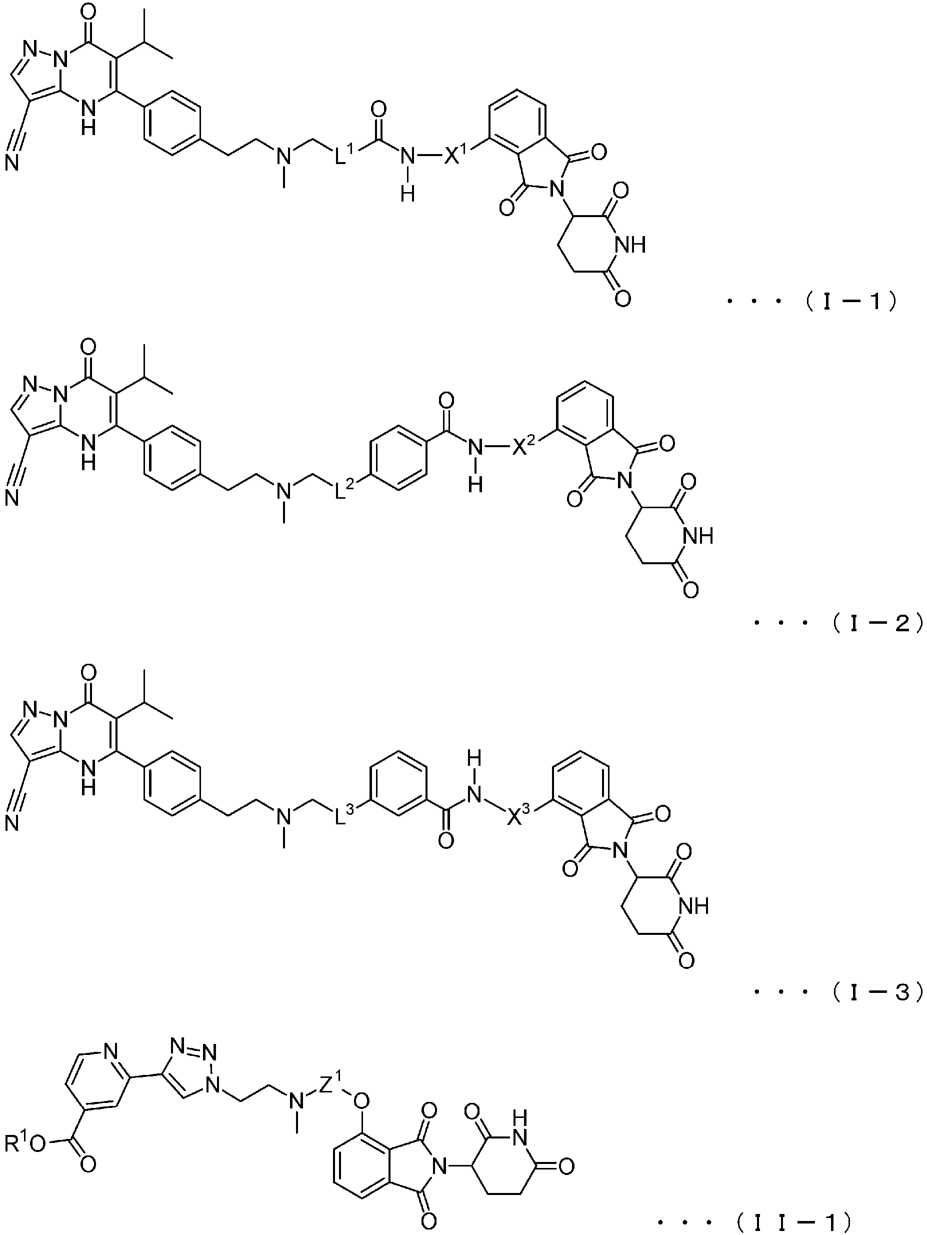
   in general formula (I-1), L¹ is a linear alkylene group having 3 to 10 carbon atoms, X¹ is a group represented by -(CH₂CH₂O)ₛ-, and s is a number from 1 to 5,
   in general formula (I-2), L² is a linear alkylene group having 3 to 10 carbon atoms, X² is a group represented by -(CH₂CH₂O)ₛ- or -(CH₂)ₜO-, s is a number from 1 to 5, and t is a number from 2 to 10,
   in general formula (I-3), L³ is a linear alkylene group having 3 to 10 carbon atoms, X³ is a group represented by -(CH₂CH₂O)ₛ- or -(CH₂)ₜO-, s is a number from 1 to 5, and t is a number from 2 to 10, and
   in general formula (II-1), R¹ is a linear alkyl group having 1 to 3 carbon atoms, and Z¹ is a substituted or unsubstituted linear alkylene group having 3 to 14 carbon atoms, or a substituted or unsubstituted branched alkylene group having 3 to 14 carbon atoms.
[6] The composition according to [5], wherein said component (A) is:
   a compound of said general formula (I-1), wherein L¹ is a linear alkylene group having 5 carbon atoms, and X¹ is a group represented by -(CH₂CH₂O)□-, and s is 3;
   a compound of said general formula (I-1), wherein L¹ is a linear alkylene group having 8 carbon atoms, and X¹ is a group represented by -(CH₂CH₂O)□-, and s is 3;
   a compound of said general formula (I-2), wherein L² is a linear alkylene group having 5 carbon atoms, and X² is a group represented by -(CH₂CH₂O)□-, and s is 3;
   a compound of said general formula (I-2), wherein L² is a linear alkylene group having 5 carbon atoms, and X² is a group represented by -(CH₂)ₜO-, and t is 6;
   a compound of said general formula (I-3), wherein L³ is a linear alkylene group having 5 carbon atoms, and X³ is a group represented by -(CH₂CH₂O)□-, and s is 3;
   a compound of said general formula (I-3), wherein L³ is a linear alkylene group having 5 carbon atoms, and X³ is a group represented by -(CH₂)ₜO-, and t is 6; or
   a compound of said general formula (II-1), wherein R¹ is a methyl group, and Z¹ is an unsubstituted linear alkylene group having 9 carbon atoms.
[7] The composition according to [1], wherein said composition is composition (1), and
   said component (B) comprises at least one component selected from the group consisting of tranylcypromine and a salt thereof.
[8] The composition according to [6], wherein said composition is composition (1), and
   said component (B) comprises at least one component selected from the group consisting of tranylcypromine and a salt thereof.
[9] A medium comprising the composition according to any one of [1] to [8].
[10] The medium according to [9], wherein said medium is a medium for culturing human hematopoietic stem cells.
[11] The medium according to [9], wherein said medium is a serum-free medium.
[12] The medium according to [9], wherein said medium is an albumin-free medium.
[13] The medium according to [9], wherein said medium comprises at least one cell type selected from the group consisting of CD34⁺ cells, CD201⁺ cells, CD90⁺ cells, and CD41⁺ cells.
[14] The medium according to [9], wherein said medium comprises a cell population enriched with at least one cell type selected from the group consisting of CD201⁺ cells, CD90⁺ cells, and CD41⁺ cells.
[15] A composition used for culturing cells obtained by culturing in the medium according to [9].
[16] The composition according to [15], wherein the cells obtained by culturing in said medium are human hematopoietic stem cells.
[17] The composition according to [15], wherein the cells obtained by culturing in said medium are CD201⁺ or CD41⁺ cells.
[18] A cell population obtained by culturing in the medium according to [9].
[19] The cell population according to [18], wherein said cell population comprises human hematopoietic stem cells.
[20] The cell population according to [18], wherein said cell population comprises CD201⁺ or CD41⁺ cells.
[21] At least one therapeutic agent selected from the group consisting of a cancer therapeutic agent, an immunodeficiency therapeutic agent, and a multiple myeloma therapeutic agent, comprising cells obtained by culturing in the medium according to [9].
[22] A method for producing cells, comprising a step of culturing cells using the medium according to [9].
[23] The method for producing cells according to [22], wherein said culturing step comprises obtaining a medium from said cells and said composition.
[24] The method for producing cells according to [22], comprising a step of culturing the cells cultured in said culturing step using another medium.
[25] The method for producing cells according to [22], wherein said cultured cells are a cell population comprising human hematopoietic stem cells.
[26] The method for producing cells according to [22], wherein said cultured cells are a cell population comprising CD201⁺ or CD41⁺ human hematopoietic stem cells.
[27] A cell population produced by the method according to [22].
[28] The cell population according to [27], wherein said cell population comprises human hematopoietic stem cells.
[29] The cell population according to [27], wherein said cell population comprises CD201⁺ or CD41⁺ cells.
[30] At least one therapeutic agent selected from the group consisting of a cancer therapeutic agent, an immunodeficiency therapeutic agent, and a multiple myeloma therapeutic agent, comprising cells obtained using the method according to [22].
[31] Use of an agent comprising at least one component selected from the group consisting of a compound represented by the following formula (I) and a salt thereof, and a compound represented by the following general formula (II) and a salt thereof, for the manufacture of a medium for expansion of hematopoietic stem cells: 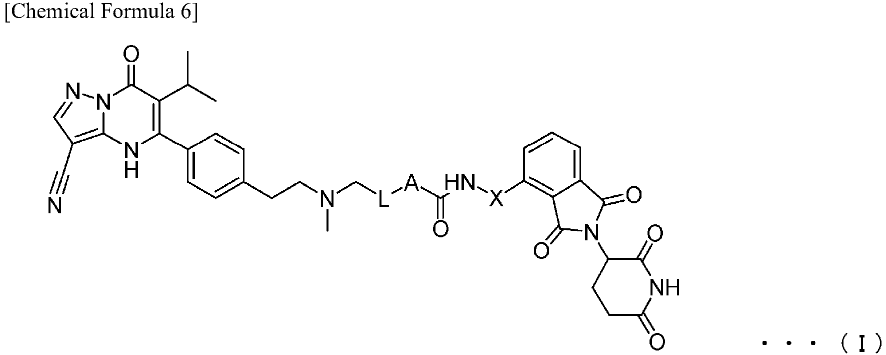 in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10; 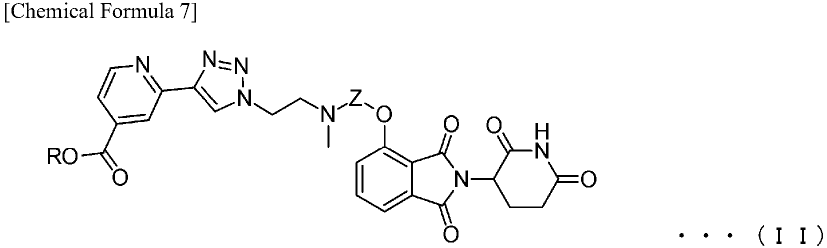 in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.
[32] Use of an agent comprising at least one component selected from the group consisting of a compound represented by the following general formula (I) and a salt thereof, and a compound represented by the following general formula (II) and a salt thereof, as an agent for a medium for expansion of hematopoietic stem cells: 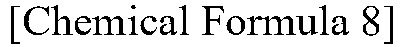 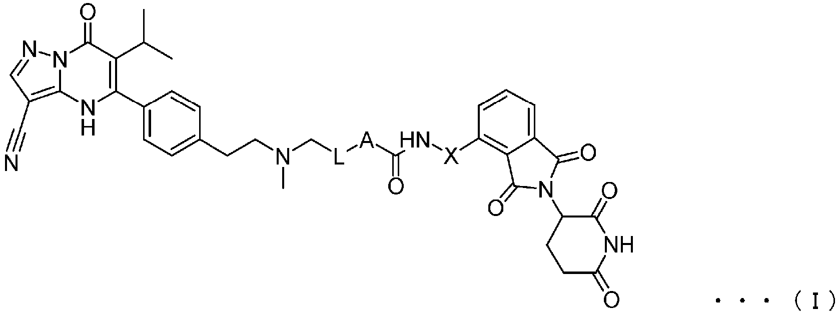 in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)ₛ-, - (CH₂CH₂CH₂O)ₛ-, -(CH(CH₃)CH₂O)ₛ-, or -(CH₂)ₜO-, s is a number from 1 to 8, and t is a number from 2 to 10; 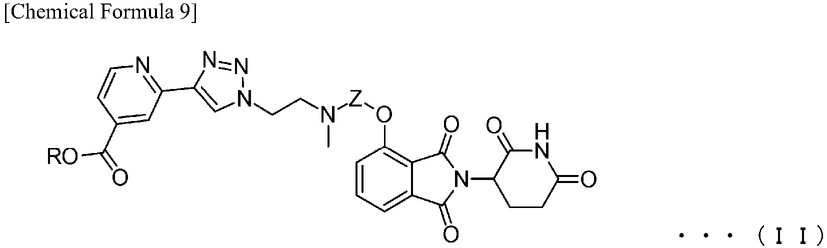 in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.
[33] Use of the composition according to any one of [1] to [8] for expansion of hematopoietic stem cells.
[34] The use according to [33], wherein said hematopoietic stem cells are intended for differentiation into megakaryocytes.
[35] Use of the composition according to any one of [1] to [8] as an agent for a medium for proliferating hematopoietic stem cells that are intended for differentiation into megakaryocytes.
[36] Use of the composition according to any one of [1] to [8] for the manufacture of an agent for a medium for expansion of hematopoietic stem cells that are intended for differentiation into megakaryocytes.
[37] The composition according to any one of [1] to [8], which is for expansion of hematopoietic stem cells.
[38] The composition according to [37], wherein said hematopoietic stem cells are intended for differentiation into megakaryocytes.
[39] The composition according to [37] or [38], wherein said composition does not contain serum.
[40] The composition according to any one of [37] to [39], wherein said composition is albumin-free.
[41] The composition according to any one of [37] to [40], wherein said composition comprises at least one cell type selected from the group consisting of CD34⁺ cells, CD201⁺ cells, CD90⁺ cells, and CD41⁺ cells.
[42] The composition according to any one of [37] to [41], wherein said composition comprises a cell population enriched with at least one cell type selected from the group consisting of CD201⁺ cells, CD90⁺ cells, and CD41⁺ cells.

### Advantageous Effects of Invention

According to the present disclosure, a composition and a medium used for cell culture can be provided.

Furthermore, according to the present disclosure, a cell population that can be transplanted into patients with diseases such as cancer and immunodeficiency, and a method for producing the same can be provided.

Furthermore, according to the present disclosure, a therapeutic agent comprising said cell population can be provided.

### BRIEF DESCRIPTION OF DRAWINGS

[FIG. 1] FIG. 1 is a diagram showing the results of expression analysis of the cell population in Example 1.
[FIG. 2] FIG. 2 is a diagram showing the results of expression analysis of the cell population in Example 2.
[FIG. 3] FIG. 3 is a diagram showing the results of expression analysis of the cell population in Example 3.
[FIG. 4] FIG. 4 is a diagram showing the results of expression analysis of the cell population in Example 4.
[FIG. 5] FIG. 5 is a diagram showing the results of expression analysis of the cell population in Example 5.
[FIG. 6] FIG. 6 is a diagram showing the results of expression analysis of the cell population in Example 6.
[FIG. 7] FIG. 7 is a diagram showing the results of expression analysis of the cell population in Example 7.
[FIG. 8] FIG. 8 is a diagram showing the results of expression analysis of the cell population in Example 8.
[FIG. 9] FIG. 9 is a diagram showing the results of expression analysis of the cell population in Example 9.
[FIG. 10] FIG. 10 is a diagram showing the results of expression analysis of the cell population in Example 10.
[FIG. 11] FIG. 11 is a diagram showing the results of expression analysis of the cell population in Example 11.
[FIG. 12] FIG. 12 is a diagram showing the results of expression analysis of the cell population in Example 12.
[FIG. 13] FIG. 13 is a diagram showing the results of expression analysis of the cell population in Example 13.
[FIG. 14] FIG. 14 is a diagram showing the results of expression analysis of the cell population in Example 14.
[FIG. 15] FIG. 15 is a diagram showing the results of expression analysis of the cell population in Example 15.
[FIG. 16] FIG. 16 is a diagram showing the results of expression analysis of the cell population in Example 16.
[FIG. 17] FIG. 17 is a diagram showing the results of expression analysis of the cell population in Example 17.
[FIG. 18] FIG. 18 is a diagram showing the results of expression analysis of the cell population in Example 18.
[FIG. 19] FIG. 19 is a diagram showing the results of expression analysis of the cell population in Example 19.
[FIG. 20] FIG. 20 is a diagram showing the results of expression analysis of the cell population in Example 20.
[FIG. 21] FIG. 21 is a diagram showing the results of expression analysis of the cell population in Example 21.
[FIG. 22] FIG. 22 is a diagram showing the results of expression analysis of the cell population in Example 22.
[FIG. 23] FIG. 23 is a diagram showing the results of expression analysis of the cell population in Example 23.
[FIG. 24] FIG. 24 is a diagram showing the results of expression analysis of the cell population in Example 24.
[FIG. 25] FIG. 25 is a diagram showing the results of expression analysis of the cell population in Example 25.
[FIG. 26] FIG. 26 is a diagram showing the results of expression analysis of the cell population in Example 26.
[FIG. 27] FIG. 27 is a diagram showing the results of expression analysis of the cell population in Example 27.
[FIG. 28] FIG. 28 is a diagram showing the results of expression analysis of the cell population in Example 28.
[FIG. 29] FIG. 29 is a diagram showing the ratio of mature megakaryocytes (CD41⁺CD42b⁺ cells) to the cell population when cultured for 7 days in Example 29.
[FIG. 30] FIG. 30 is a diagram showing the ratio of mature megakaryocytes (CD41⁺CD42b⁺ cells) to the cell population when cultured for 14 days in Example 30.

### DESCRIPTION OF EMBODIMENTS

Hereinafter, embodiments of the present disclosure will be described in detail. Note that similar content will be omitted as appropriate to avoid the complexity due to repetition.

Unless otherwise specified, "alkyl group" is intended to include linear, branched, and cyclic monovalent saturated hydrocarbon groups. The same applies to the alkyl group in an alkoxy group.

Unless otherwise specified, "alkylene group" is intended to include linear, branched, and cyclic divalent saturated hydrocarbon groups.

Unless otherwise specified, "alkenylene group" is intended to include linear, branched, and cyclic divalent unsaturated hydrocarbon groups.

"Halogen atom" includes fluorine, chlorine, bromine, and iodine atoms.

The description "substituted or unsubstituted" includes both the case of substituting a hydrogen atom (-H) with a monovalent substituent and the case of replacing a methylene group (-CH₂-) or a methine group (>CH- or =CH-) with a divalent substituent.

"Monovalent substituent" includes an alkyl group, an alkoxy group, a halogen atom, a halogenated alkyl group, a hydroxy group, a carboxy group, an amino group, a nitro group, and the like.

As the alkyl group as a substituent, an alkyl group having 1 to 5 carbon atoms is preferable, and a methyl group, ethyl group, propyl group, n-butyl group, and tert-butyl group are most preferable.

As the alkoxy group as a substituent, an alkoxy group having 1 to 5 carbon atoms is preferable, a methoxy group, ethoxy group, n-propoxy group, iso-propoxy group, n-butoxy group, and tert-butoxy group are more preferable, and a methoxy group and ethoxy group are most preferable.

As the halogen atom as a substituent, a fluorine atom and an iodine atom are preferable.

As the halogenated alkyl group as a substituent, a group in which some or all of the hydrogen atoms of an alkyl group having 1 to 5 carbon atoms, such as a methyl group, ethyl group, propyl group, n-butyl group, tert-butyl group, etc., are substituted with said halogen atom may be mentioned.

"Divalent substituent" includes -O-, -C(=O)-O-, -O-C(=O)-, -C(=O)-, -O-C(=O)-O-, -C(=O)-NH-, - C(=O)-NH-C(=O)-, -N=, -NH-, -NH-C(=NH)- (wherein H may be substituted with a substituent such as a hydrocarbon group, acyl group, alkoxyalkyl group, etc.), -S-, -S(=O)₂-, -S(=O)₂-O-, a group represented by the general formula -Y²¹-O-Y²²-, -Y²¹-O-, -Y²¹-C(=O)-O-, -C(=O)-O-Y²¹-, -[Y²¹-C(=O)-O]_{m"}-Y²²-, -Y²¹-O-C(=O)-Y²²- or -Y²¹-S(=O)₂-O-Y²²- [wherein Y²¹ and Y²² are each independently a substituted or unsubstituted divalent hydrocarbon group, O is an oxygen atom, and m" is an integer of 0 to 3], and the like.

### (1) Composition

According to one embodiment of the present disclosure, there is provided a composition (1) comprising component (A), which is a KDM5 degrader, and component (B), which is an LSD1 inhibitor, or a composition (2) comprising component (A), which is a KDM5 degrader, wherein in said composition (2), said component (A) comprises at least one component selected from the group consisting of a compound represented by the following general formula (I) and a salt thereof, and a compound represented by the following general formula (II) and a salt thereof (hereinafter, composition (1) and composition (2) are also collectively referred to simply as "composition").

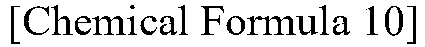

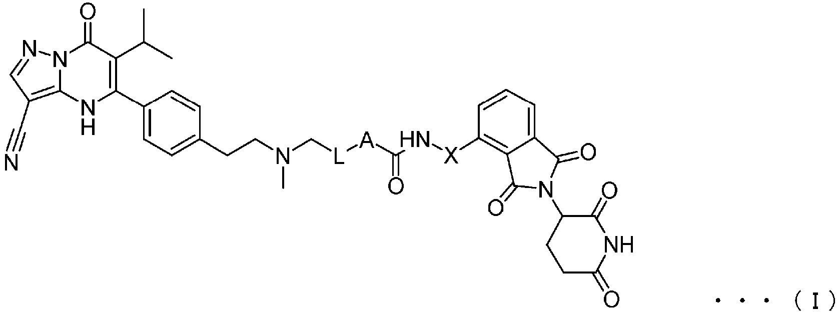

In general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)ₛ-, - (CH₂CH₂CH₂O)ₛ-, -(CH(CH₃)CH₂O)ₛ-, or -(CH₂)ₜO-, s is a number from 1 to 8, and t is a number from 2 to 10.

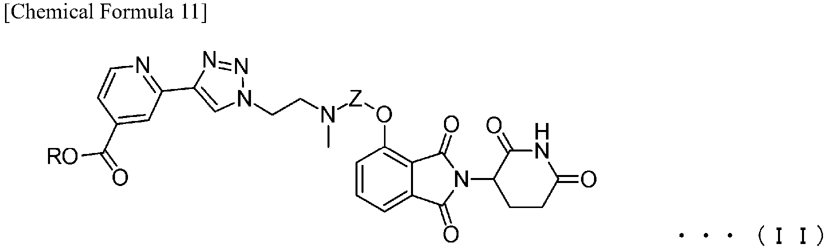

In general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

In general formula (I), A is a single bond or a substituted or unsubstituted divalent aromatic hydrocarbon group, L is a substituted or unsubstituted linear or branched alkylene group having 1 to 13 carbon atoms, X is a group represented by -(CH₂CH₂O)ₛ-, -(CH₂CH₂CH₂O)ₛ-, -(CH(CH₃)CH₂O)ₛ-, or -(CH₂)ₜO-, s is a number from 1 to 8, and t is a number from 2 to 10.

Said A is preferably a single bond, phenylene group, naphthylene group, or the like; more preferably a single bond, 1,2-phenylene group, 1,3-phenylene group (m-phenylene group), 1,4-phenylene group (p-phenylene group), 1,2-naphthylene group, 1,4-naphthylene group, 1,5-naphthylene group, 2,3-naphthylene group, 2,6-naphthylene group, or the like; and still more preferably a single bond, 1,3-phenylene group (m-phenylene group), and 1,4-phenylene group (p-phenylene group).

Said L is preferably a linear alkylene group having 3 to 10 carbon atoms, more preferably a linear alkylene group having 4 to 9 carbon atoms, and still more preferably a linear alkylene group having 5 to 8 carbon atoms.

Said X is preferably a group represented by -(CH₂CH₂O)□, -(CH₂CH₂CH₂O)□-, -(CH(CH₃)CH₂O)□-, or - (CH₂)ₜO-; and more preferably a group represented by -(CH₂CH₂O)□-, -(CH₂CH₂CH₂O)□-, or -(CH₂)ₜO-.

Said s is preferably a number from 1 to 5, more preferably 2 to 4, and still more preferably 2 or 3.

Said t is preferably a number from 2 to 10, more preferably 3 to 8, and still more preferably 4 to 7.

In said general formula (I), it is preferable that A is a single bond or phenylene group, L is a linear alkylene group having 3 to 10 carbon atoms, X is a group represented by -(CH₂CH₂O)□-, -(CH₂CH₂CH₂O)□-, - (CH(CH₃)CH₂O)□, or -(CH₂)ₜO-, s is a number from 1 to 5, and t is a number from 2 to 10.

It is more preferable that in said general formula (I), A is a single bond, p-phenylene group, or m-phenylene group, L is a linear alkylene group having 3 to 10 carbon atoms, X is a group represented by - (CH₂CH₂O)□-, -(CH₂CH₂CH₂O)□-, or -(CH₂)ₜO-, s is a number from 1 to 5, and t is a number from 2 to 10.

In general formula (II), said R is preferably a linear or branched alkyl group having 1 to 6 carbon atoms, more preferably a linear or branched alkyl group having 1 to 3 carbon atoms, still more preferably a methyl or ethyl group, and particularly preferably a methyl group.

Said Z is preferably a linear or branched alkylene group having 2 to 20 carbon atoms, more preferably a linear or branched alkylene group having 3 to 18 carbon atoms, still more preferably a linear or branched alkylene group having 5 to 15 carbon atoms, particularly preferably an n-hexylene group, n-heptylene group, n-octylene group, n-nonylene group, n-decylene group, or n-undecylene group, and most preferably an n-octylene group, n-nonylene group, or n-decylene group.

In said composition (1),
it is preferable that said component (A) comprises at least one component selected from the group consisting of a compound represented by the following general formula (I) and a salt thereof, and a compound represented by the following general formula (II) and a salt thereof.

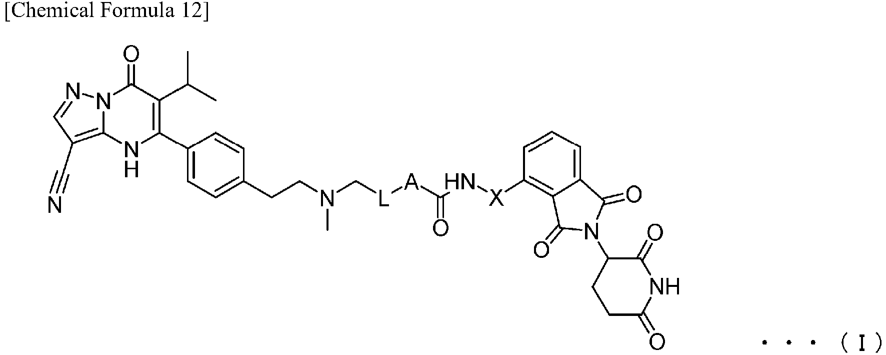

In general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10.

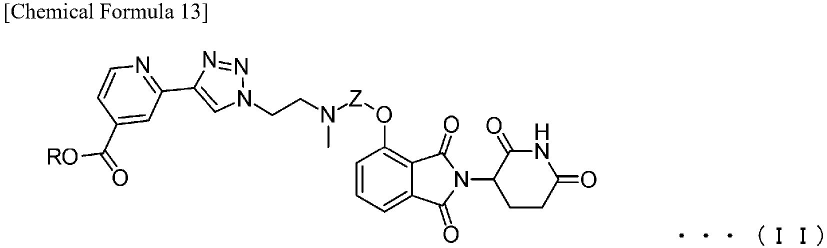

In general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

It is preferable for the composition that
in said general formula (I), A is a single bond or a phenylene group, L is a linear alkylene group having 3 to 10 carbon atoms, X is a group represented by -(CH₂CH₂O)□-, -(CH₂CH₂CH₂O)□-, -(CH(CH₃)CH₂O)□-, or -(CH₂)ₜO-, s is a number from 1 to 5, and t is a number from 2 to 10, and
in said general formula (II), R is a hydrogen atom, or a linear alkyl group having 1 to 6 carbon atoms, and Z is a substituted or unsubstituted linear alkylene group having 2 to 18 carbon atoms, or a substituted or unsubstituted branched alkylene group having 2 to 18 carbon atoms.

It is preferable for the composition that
in said general formula (I), A is a single bond, p-phenylene group, or m-phenylene group, L is a linear alkylene group having 3 to 10 carbon atoms, X is a group represented by -(CH₂CH₂O)□-, -(CH₂CH₂CH₂O)□-, or - (CH₂)ₜO-, s is a number from 1 to 5, and t is a number from 2 to 10, and
in said general formula (II), R is a linear alkyl group having 1 to 6 carbon atoms, and Z is a substituted or unsubstituted linear alkylene group having 3 to 16 carbon atoms, or a substituted or unsubstituted branched alkylene group having 3 to 16 carbon atoms.

It is preferable for the composition that said component (A) is at least one selected from the group consisting of a compound represented by the following general formula (I-1) and a salt thereof, a compound represented by the following general formula (I-2) and a salt thereof, a compound represented by the following general formula (I-3) and a salt thereof, and a compound represented by the following general formula (II-1) and a salt thereof.

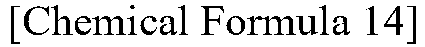

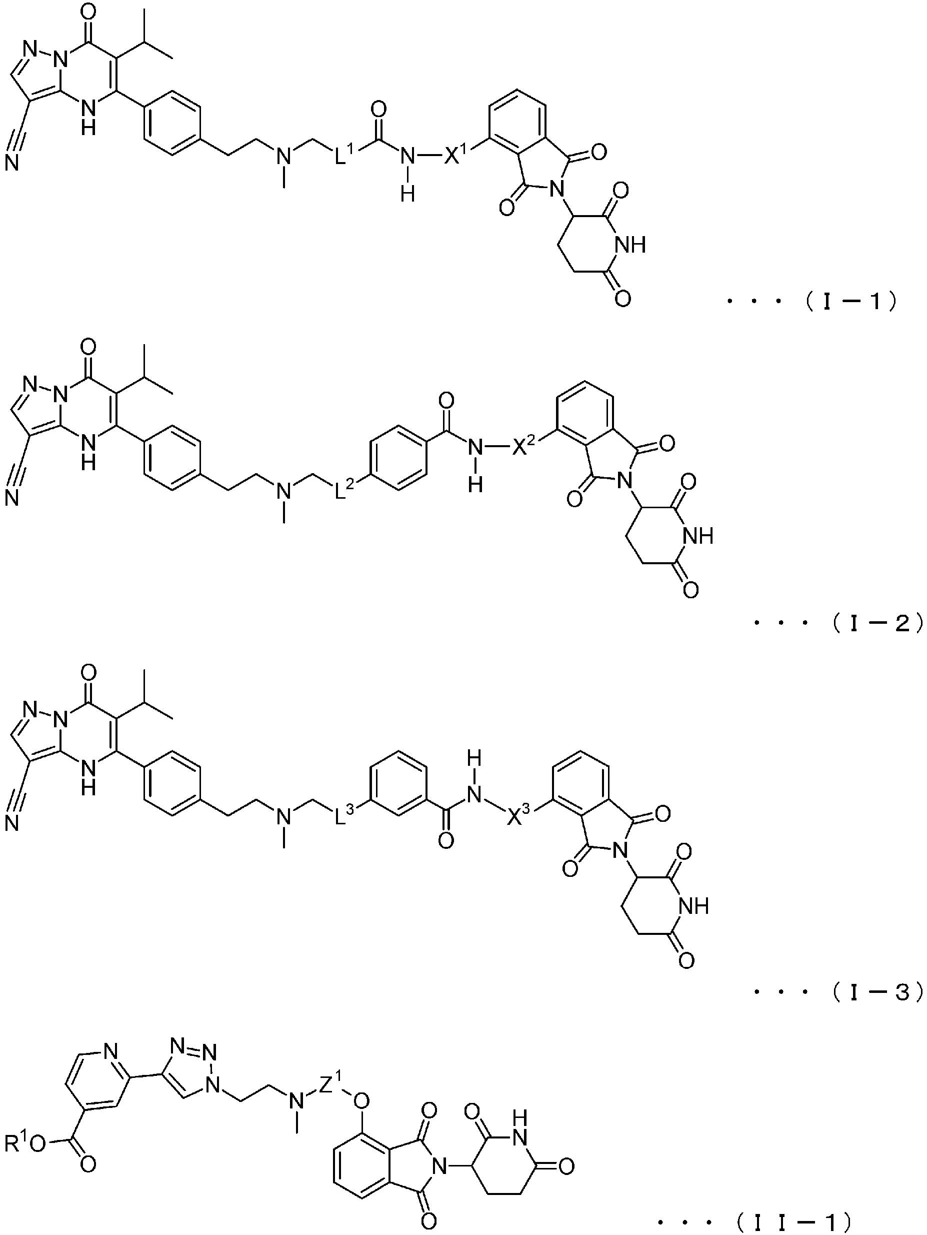

It is preferable that in general formula (I-1), L¹ is a linear alkylene group having 3 to 10 carbon atoms, X¹ is a group represented by -(CH₂CH₂O)ₛ-, and s is a number from 1 to 5, in general formula (I-2), L² is a linear alkylene group having 3 to 10 carbon atoms, X² is a group represented by -(CH₂CH₂O)ₛ- or -(CH₂)ₜO-, s is a number from 1 to 5, and t is a number from 2 to 10, and in general formula (I-3), L³ is a linear alkylene group having 3 to 10 carbon atoms, X³ is a group represented by -(CH₂CH₂O)ₛ- or -(CH₂)ₜO-, s is a number from 1 to 5, and t is a number from 2 to 10.

It is preferable that in general formula (II-1), R¹ is a linear alkyl group having 1 to 3 carbon atoms, and Z¹ is a substituted or unsubstituted linear alkylene group having 3 to 14 carbon atoms, or a substituted or unsubstituted branched alkylene group having 3 to 14 carbon atoms.

It is preferable that in the composition, said component (A) is:
a compound of said general formula (I-1), wherein L¹ is a linear alkylene group having 5 carbon atoms, and X¹ is a group represented by -(CH₂CH₂O)□-, and s is 3;
a compound of said general formula (I-1), wherein L¹ is a linear alkylene group having 8 carbon atoms, and X¹ is a group represented by -(CH₂CH₂O)□-, and s is 3;
a compound of said general formula (I-2), wherein L² is a linear alkylene group having 5 carbon atoms, and X² is a group represented by -(CH₂CH₂O)□-, and s is 3;
a compound of said general formula (I-2), wherein L² is a linear alkylene group having 5 carbon atoms, and X² is a group represented by -(CH₂)ₜO-, and t is 6;
a compound of said general formula (I-3), wherein L³ is a linear alkylene group having 5 carbon atoms, and X³ is a group represented by -(CH₂CH₂O)□-, and s is 3;
a compound of said general formula (I-3), wherein L³ is a linear alkylene group having 5 carbon atoms, and X³ is a group represented by -(CH₂)ₜO-, and t is 6; or
a compound of said general formula (II-1), wherein R¹ is a methyl group, and Z¹ is an unsubstituted linear alkylene group having 9 carbon atoms.

Among them, as the compound represented by general formula (I) and a salt thereof, a compound wherein in formula (I), A is an m-phenylene group, L is an n-pentylene group (a linear alkylene group having 5 carbon atoms), and X is a group represented by -(CH₂)ₜO-, and t is 6 (hereinafter, also referred to as "compound 126") or a salt thereof is preferable. As the compound represented by general formula (II) and a salt thereof, a compound wherein R is a methyl group, and Z is an n-nonylene group (a linear alkylene group having 9 carbon atoms) (hereinafter, also referred to as "compound 135") or a salt thereof is preferable.

It is preferable that said composition is composition (1), and said component (B) is comprising at least one component selected from the group consisting of tranylcypromine represented by the following general formula (B-1) or (B-2) and a salt thereof. Among them, as tranylcypromine and a salt thereof, tranylcypromine represented by the following general formula (B-2) and a salt thereof are more preferable, and tranylcypromine hydrochloride represented by the following general formula (B-2-1) is still more preferable.

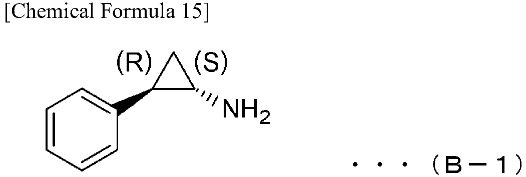

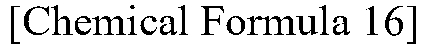

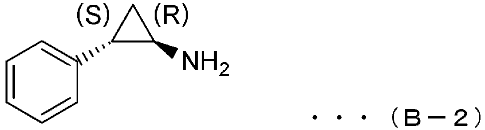

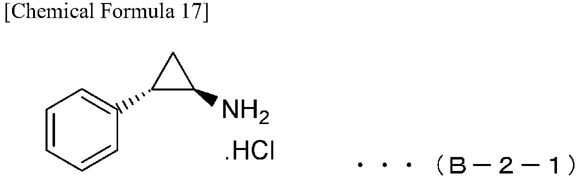

Salts of said compound include all pharmacologically acceptable salts. All pharmacologically acceptable salts are preferably low-toxicity water-soluble salts. Examples of suitable salts include acid addition salts (e.g., inorganic acid salts [e.g., hydrochloride, hydrobromide, hydroiodide, sulfate, phosphate, nitrate, etc.], organic acid salts [e.g., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, etc.], salts with acidic natural amino acids [e.g., aspartate, glutamate, etc.]), and the like.

The compound represented by said general formula (I) can be produced by a method similar to the production method described in Japanese Patent Application No. 2023-143583.

The compound represented by said general formula (II) can be produced by a method similar to the production method described in ChemMedChem. 2021 May 18; 16(10): 1609-1618.

Said composition (1) can be produced by mixing component (A), a KDM5 degrader, and component (B), an LSD1 inhibitor, and optionally other arbitrary base components.

Said composition (2) can be produced by mixing, as component (A), a KDM5 degrader, at least one component selected from the group consisting of a compound represented by the above general formula (I) and a salt thereof, and a compound represented by the above general formula (II) and a salt thereof, and optionally other components.

Other components include those described below as arbitrary base components.

In said composition (1), the content of compound (I) of said component (A) is preferably 0.1 to 100 µmol/L, more preferably 0.3 to 30 µmol/L, and still more preferably 1 to 10 µmol/L, with respect to the total amount (1 L) of the composition.

In said composition (1), the content of compound (II) of said component (A) is preferably 0.1 to 100 µmol/L, more preferably 0.3 to 30 µmol/L, and still more preferably 1 to 10 µmol/L, with respect to the total amount (1 L) of the composition.

In said composition (1), the content of said component (B) is preferably 0.1 to 100 µmol/L, more preferably 0.3 to 30 µmol/L, and still more preferably 1 to 10 µmol/L, with respect to the total amount (1 L) of the composition.

In said composition (2), the content of compound (I) of said component (A) is preferably 0.1 to 100 µmol/L, more preferably 0.3 to 30 µmol/L, and still more preferably 1 to 10 µmol/L, with respect to the total amount (1 L) of the composition.

In said composition (2), the content of compound (II) of said component (A) is preferably 0.1 to 100 µmol/L, more preferably 0.3 to 30 µmol/L, and still more preferably 1 to 10 µmol/L, with respect to the total amount (1 L) of the composition.

When said composition contains albumin, the content of albumin is preferably 0.1 to 20% by mass, more preferably 0.5 to 10% by mass, and still more preferably 1 to 4% by mass, with respect to the total mass of the composition.

Here, the composition may be a medium. Said medium may be a medium for culturing human hematopoietic stem cells. Said composition may further comprise hematopoietic stem cells. By using this composition, a cultured fraction containing abundant CD201⁺ or CD90⁺ cells can be efficiently produced. For example, in the Examples described below, it is described that as a result of culturing human CB CD34⁺ cells using this composition, a cultured fraction enriched with CD201⁺ cells or CD90⁺ cells was obtained. Furthermore, according to one embodiment of the present disclosure, a method for producing cells, a method for culturing cells, a method for producing a medium, and a method for enriching CD201⁺ or CD90⁺ cells utilizing this composition, as well as a container containing this composition are provided.

### (2) Medium

According to one embodiment of the present disclosure, a medium comprising the composition of (1) above is provided. By using this medium, a cultured fraction containing abundant CD201⁺ or CD90⁺ cells can be efficiently produced.

Said medium is preferably a medium for culturing human hematopoietic stem cells.

When human CB CD34⁺ cells are cultured, a cultured fraction containing abundant CD201⁺ or CD90⁺ cells can be efficiently produced, so it is preferable as a medium for culturing human hematopoietic stem cells.

When human CB CD41⁺ cells are cultured, a cultured fraction containing abundant CD41⁺ cells can be efficiently produced, so it is preferable as a medium for human hematopoietic stem cells intended for differentiation into human megakaryocytes or human platelets. Furthermore, when human CB CD41⁺34⁺ cells are cultured, a cultured fraction containing abundant cells that are both CD41⁺ and CD34⁺ can be efficiently produced, so it is more preferable as a medium for human hematopoietic stem cells intended for differentiation into human megakaryocytes or human platelets. Not limited to CB CD41⁺ cells, megakaryocytes or platelets may be differentiated from hematopoietic stem cells derived from peripheral blood, bone marrow, or pluripotent stem cells (including iPS cells and embryonic stem cells).

Said medium is preferably a serum-free medium.

Said medium is preferably an albumin-free medium.

Said medium preferably comprises at least one cell type selected from the group consisting of CD34⁺ cells, CD201⁺ cells, CD90⁺ cells, and CD41⁺ cells.

Comprising at least one cell type selected from the group consisting of CD34⁺ cells, CD201⁺ cells, and CD90⁺ cells makes it preferable as a medium for expansion of human hematopoietic stem cells.

Comprising at least one cell type selected from the group consisting of CD41⁺ cells makes it preferable as a medium for human hematopoietic stem cells intended for differentiation into human megakaryocytes or human platelets.

It is preferable that said medium comprises a cell population enriched with at least one cell type selected from the group consisting of CD201⁺ cells and CD90⁺ cells. This makes it preferable as a medium for expansion of human hematopoietic stem cells.

It is preferable that said medium comprises a cell population enriched with CD41⁺ cells. This makes it preferable as a medium for human hematopoietic stem cells intended for differentiation into human megakaryocytes or human platelets.

Cells obtained by culturing in said medium may be used for culturing in a separately prepared, unused said medium.

The cell population obtained by culturing in said medium is preferably human hematopoietic stem cells, a cell population comprising CD201⁺ cells is preferable for expansion of human hematopoietic stem cells, and a cell population comprising CD41⁺ cells is preferable for expansion of human hematopoietic stem cells that are intended for differentiation into human megakaryocytes or human platelets.

It is preferable that said medium comprises said cells and said composition.

It is preferable that said medium is obtained from said cells and said composition.

It is preferable that said medium comprises at least one type of hematopoietic stem cells selected from the group consisting of CD201⁺ cells and CD90⁺ cells, and the number of said hematopoietic stem cells after culturing for 10 days using said composition is greater than the number of said hematopoietic stem cells at the start of culturing using said composition.

It is preferable that said medium comprises human hematopoietic stem cells comprising CD41⁺ cells, and the number of said human hematopoietic stem cells after culturing for 10 days using said composition is greater than the number of said human hematopoietic stem cells at the start of culturing using said composition.

Note that the culture period is not limited to 10 days and may range from several days (about 5 days) to about 3 weeks.

It is preferable that said medium comprises at least one type of hematopoietic stem cells selected from the group consisting of CD201⁺ cells and CD90⁺ cells, and the number of cells calculated by [number of CD201⁺ cells after culturing for 10 days using said composition] - [number of CD201⁺ cells at the start of culturing using said composition] is greater than the number of cells calculated by [number of CD90⁺ cells after culturing for 10 days using said composition] - [number of CD90⁺ cells at the start of culturing using said composition].

It is preferable that said medium comprises at least one type of hematopoietic stem cells selected from the group consisting of CD41⁺ cells, and the ratio represented by [number of CD41⁺ cells after culturing for 10 days using said composition] / [number of CD41⁺ cells at the start of culturing using said composition] is 10 or more. Since the total number of cells increases 10-fold or more in 10 days of culture, a larger number of CD41⁺ cells can be obtained.

Note that in this specification, the number of cells can be measured using the flow cytometry method.

### (3) Method

According to one embodiment of the present disclosure, a method comprising a step of contacting the composition of (1) above with cells is provided. This method includes, for example, a method for producing cells, a method for culturing cells, and a method for enriching CD201⁺, CD90⁺, or CD41⁺ cells.

### (4) Production Method

According to one embodiment of the present disclosure, a method for producing cells, comprising a culturing step of culturing cells using a medium comprising the composition of (1) above, is provided. Said cells may be human cells. By using this method, a cultured fraction containing abundant CD201⁺, CD90⁺, or CD41⁺ cells can be efficiently produced.

Said method for producing cells may include a step of obtaining a medium from the composition of (1) above and said cells, before said culturing step.

### (5) Production Method

According to one embodiment of the present disclosure, a method for producing cells, comprising a culturing step of culturing CB CD34⁺ cells using a medium comprising the composition of (1) above, is provided. By using this method, a cultured fraction containing abundant CD201⁺ or CD90⁺ cells can be efficiently produced.

According to another embodiment of the present disclosure, a method for producing cells, comprising a culturing step of culturing CB CD41⁺ cells using a medium comprising the composition of (1) above, is provided. By using this method, a cultured fraction containing abundant CD41⁺ or CD34⁺ cells can be efficiently produced.

### (6) Production Method

According to one embodiment of the present disclosure, a method for producing cells, comprising a culturing step of culturing CD201⁺ or CD90⁺ cells using a medium comprising the composition of (1) above, is provided. By using this method, CD201⁺ or CD90⁺ cells can be successfully expanded.

According to another embodiment of the present disclosure, a method for producing cells, comprising a culturing step of culturing CD41⁺ cells using a medium comprising the composition of (1) above, is provided. By using this method, CD41⁺ or CD34⁺ cells can be successfully expanded.

### (7) Production Method

According to one embodiment of the present disclosure, a method for producing cells, comprising a culturing step of culturing human hematopoietic stem cells using a medium comprising the composition of (1) above, is provided. By using this method, human hematopoietic stem cells can be successfully expanded.

According to one embodiment of the present disclosure, a method for producing cells, comprising a culturing step of culturing human megakaryocytes or human platelets using a medium comprising the composition of (1) above, is provided. By using this method, human megakaryocytes or human platelets can be successfully expanded.

### (8) Production Method

According to one embodiment of the present disclosure, a method for producing the composition of (1) above and a method for producing a medium are provided. By using the medium obtained by this method, a cultured fraction containing abundant CD201⁺ or CD90⁺ cells can be efficiently produced.

According to another embodiment of the present disclosure, a method for producing the composition of(1) above and a method for producing a medium are provided. By using the medium obtained by this method, a cultured fraction containing abundant CD41⁺ or CD34⁺ cells can be efficiently produced.

### (9) Production Method

According to one embodiment of the present disclosure, a method for producing a cell population is provided, the method comprising a step of sorting hematopoietic stem cell marker⁺ cells from a composition comprising a population of cells obtained by any of the methods described in (3) to (8) above, a step of culturing the sorted hematopoietic stem cell marker⁺ cells, or a step of collecting the cultured hematopoietic stem cell marker⁺ cells. By using this method, hematopoietic stem cells can be further enriched, or a larger number of hematopoietic stem cells can be collected.

Furthermore, by using this method, hematopoietic stem cells intended for differentiation into megakaryocytes or platelets can be further enriched, or a larger number of hematopoietic stem cells intended for differentiation into megakaryocytes or platelets can be collected.

### (10) Culture Method

According to one embodiment of the present disclosure, a method for culturing cells, comprising a culturing step of culturing cells in a medium comprising the composition of (1) above, is provided. By using this method, a cultured fraction containing abundant CD201⁺ or CD90⁺ cells can be efficiently produced.

According to another embodiment of the present disclosure, a method for culturing cells, comprising a culturing step of culturing cells in a medium comprising the composition of (1) above, is provided. By using this method, a cultured fraction containing abundant CD41⁺ or CD34⁺ cells can be efficiently produced.

### (11) Enrichment Method

According to one embodiment of the present disclosure, a method for enriching CD201⁺ or CD90⁺ cells, comprising a culturing step of culturing cells in a medium comprising the composition of (1) above, is provided. By using this method, a cultured fraction containing abundant CD201⁺ or CD90⁺ cells can be efficiently produced.

According to one embodiment of the present disclosure, a method for enriching CD41⁺ or CD34⁺ cells, comprising a culturing step of culturing cells in a medium comprising the composition of (1) above, is provided. By using this method, a cultured fraction containing abundant CD41⁺ or CD34⁺ cells can be efficiently produced.

### (12) Enrichment Method

According to one embodiment of the present disclosure, a method for enriching hematopoietic stem cells, comprising a culturing step of culturing cells in a medium comprising the composition of (1) above, is provided. By using this method, a cultured fraction containing abundant hematopoietic stem cells can be efficiently produced.

### (13) Container

According to one embodiment of the present disclosure, a container comprising the composition of (1) above is provided. By using this container, a cultured fraction containing abundant CD201⁺ or CD90⁺ cells can be efficiently produced.

According to one embodiment of the present disclosure, a container comprising the composition of (1) above is provided. By using this container, a cultured fraction containing abundant CD41⁺ or CD34⁺ cells can be efficiently produced.

### (14) Container

According to one embodiment of the present disclosure, a container comprising cells or a cell population thereof, obtained by any of the methods described in (3) to (12) above, is provided. This container can be used for hematopoietic stem cell transplantation. Before hematopoietic stem cell transplantation, a step of sorting desired cells (e.g., hematopoietic stem cell marker⁺ cells) from the cell population, a step of culturing the sorted cells, a step of collecting the cultured cells, or a step of maintaining the collected cells in a container may be performed.

### (15) Cells

According to one embodiment of the present disclosure, cells or a cell population thereof, obtained by any of the methods described in (3) to (12) above, are provided. These cells or this cell population can be used for hematopoietic stem cell transplantation. Before hematopoietic stem cell transplantation, a step of sorting desired cells (e.g., hematopoietic stem cell marker⁺ cells) from the cell population, a step of culturing the sorted cells, a step of collecting the cultured cells, or a step of maintaining the collected cells in a container may be performed.

### (16) Cells

According to one embodiment of the present disclosure, a cell population comprising 5% or more of CD201⁺ or CD90⁺ cells is provided. This cell population can be used for the production of a pharmaceutical composition for hematopoietic stem cell transplantation.

According to one embodiment of the present disclosure, a cell population comprising 5% or more of CD41⁺ or CD34⁺ cells is provided. This cell population can be used for the production of a pharmaceutical composition for hematopoietic stem cell transplantation.

Said cell population preferably comprises human hematopoietic stem cells, and is preferably a cell population comprising CD201⁺ or CD41⁺ cells.

It is preferable that said cell population comprises at least one cell type selected from the group consisting of CD34⁺ cells, CD201⁺ cells, CD90⁺ cells, and CD41⁺ cells.

It is preferable that said cell population comprises a cell population enriched with at least one cell type selected from the group consisting of CD201⁺ cells, CD90⁺ cells, and CD41⁺ cells.

A cell population obtained by culturing in said medium may be used for culturing in a separately prepared, unused said medium.

It is preferable that said cell population comprises at least one hematopoietic stem cell type selected from the group consisting of CD201⁺ cells, CD90⁺ cells, and CD41⁺ cells, and the number of said hematopoietic stem cells after culturing for 10 days using said composition is greater than the number of said hematopoietic stem cells at the start of culturing using said composition.

It is preferable that said cell population comprises at least one hematopoietic stem cell type selected from the group consisting of CD201⁺ cells and CD90⁺ cells, and the number of cells calculated by [number of CD201⁺ cells after culturing for 10 days using said composition] - [number of CD201⁺ cells at the start of culturing using said composition] is greater than the number of cells calculated by [number of CD90⁺ cells after culturing for 10 days using said composition] - [number of CD90⁺ cells at the start of culturing using said composition].

### (17) Medium

According to one embodiment of the present disclosure, a cell-containing medium is provided, wherein the proportion of CD201⁺, CD90⁺ or CD41⁺ cells to the total cells in the medium is 5% or more. This medium can be used for the production of a pharmaceutical composition for hematopoietic stem cell transplantation.

### (18) Composition

According to one embodiment of the present disclosure, the composition of (1) above for use in any of the methods described in (3) to (12) above is provided.

### (19) Composition

According to one embodiment of the present disclosure, the cells or cell population thereof according to (15) or (16) above and the composition of (1) above are provided. Since this composition contains abundant CD201⁺, CD90⁺ or CD41⁺ cells, it is useful for producing a pharmaceutical composition for use in hematopoietic stem cell transplantation.

### (20) Composition

According to one embodiment of the present disclosure, a pharmaceutical composition for use in hematopoietic stem cell transplantation, comprising cells or a cell population thereof obtained by any of the methods described in (3) to (12) above, or a method for producing the same, is provided. This production method may include, for example, a step of purifying hematopoietic stem cells from the cell population described in (15) or (16) above. This production method may further include, for example, a step of sorting hematopoietic stem cell marker⁺ cells from the composition described in (19) above, a step of culturing the sorted hematopoietic stem cell marker⁺ cells, or a step of collecting the cultured hematopoietic stem cell marker⁺ cells. From another aspect, according to one embodiment of the present disclosure, the cells or cell population according to (15) or (16) above, or a pharmaceutical composition comprising said cells or cell population, for use in hematopoietic stem cell transplantation, is provided. From another aspect, according to one embodiment of the present disclosure, the use of the cells or cell population according to (15) or (16) above in the production of a pharmaceutical composition for hematopoietic stem cell transplantation is provided.

### (21) Transplantation Method

According to one embodiment of the present disclosure, a transplantation method is provided. This transplantation method may include, for example, a step of transplanting the cells or cell population according to (15) or (16) above, or a pharmaceutical composition comprising said cells or cell population, into a subject. According to this transplantation method, hematopoietic stem cells can be effectively engrafted, thereby treating a disease.

### (22) Method of Using Cell Population

According to one embodiment of the present disclosure, at least one therapeutic agent selected from the group consisting of a cancer therapeutic agent, an immunodeficiency therapeutic agent, and a multiple myeloma therapeutic agent, comprising said cell population, can be provided. This therapeutic agent can be used, for example, by transplanting it into a subject according to the transplantation method of (21) above. According to this therapeutic agent, hematopoietic stem cells can be effectively engrafted, thereby treating a disease.

### (23) Method of Using Compound

According to one embodiment of the present disclosure, an agent comprising at least one compound selected from the group consisting of a compound represented by the above general formula (I) and a compound represented by the above general formula (II) can be used for producing a medium for expansion of hematopoietic stem cells.

According to one embodiment of the present disclosure, an agent comprising at least one compound selected from the group consisting of a compound represented by the above general formula (I) and a compound represented by the above general formula (II) can be used as an agent for a medium for expansion of hematopoietic stem cells.

It is preferable that the agent comprises at least one compound selected from the group consisting of compound 126 and compound 135.

Other components that may be included in the agent include those described below as arbitrary base components.

In an embodiment of the present disclosure (including, for example, (3) to (12) or (21) above), the method may include one or more of the following steps (i) to (v): (i) a step of placing the composition of (1) above in a container or maintaining it in a container before culturing, (ii) a step of contacting said cells with said composition in a container, (iii) a step of culturing the cells in a container, (iv) a step of generating a cell population enriched with CD201⁺, CD90⁺ or CD41⁺ cells, or (v) a step of generating a cell-containing medium in which the proportion of CD201⁺, CD90⁺ or CD41⁺ cells to the total cells in the medium is 5% or more. The method of an embodiment of the present disclosure (including, for example, (3) to (12) or (21) above) may include one or more of the following steps (vi) to (xx): (vi) a step of preparing a cell population comprising hematopoietic stem cells, (vii) a step of preparing a cell population comprising CB CD34⁺ cells, (viii) a step of sorting CD34⁺ cells from the cell population, (ix) a step of culturing the sorted CD34⁺ cells, (x) a step of producing the composition of (1) above before culturing, (xi) a step of incubating the cells in a culture medium, (xii) a step of seeding and incubating the cells in a medium in a container, (xiii) a step of sorting or collecting CD201⁺, CD90⁺ or CD41⁺ cells from the cell population, (xiv) a step of sorting or collecting hematopoietic stem cells from the cell population, (xv) a step of culturing the sorted or collected CD201⁺, CD90⁺ or CD41⁺ cells, (xvi) a step of culturing the sorted or collected hematopoietic stem cells, (xvii) a step of collecting the sorted or collected and cultured CD201⁺, CD90⁺ or CD41⁺ cells, (xviii) a step of collecting the sorted or collected and cultured hematopoietic stem cells, (xix) a step of preserving (e.g., cryopreserving) the collected CD201⁺, CD90⁺ or CD41⁺ cells, or (xx) a step of transplanting the collected CD201⁺, CD90⁺ or CD41⁺ cells into a subject. Employing one or more of these steps is useful for efficiently producing a cultured fraction containing abundant CD201⁺, CD90⁺ or CD41⁺ cells. When two or more steps are employed from the embodiments of the present disclosure (including, for example, the methods of (3) to (12), (21), or the steps of (i) to (xx) above), the order is arbitrary and can be determined according to the desired operation. Each method or step of the embodiments of the present disclosure (including, for example, the methods of (3) to (12), (21), or the steps of (i) to (xx) above) may be performed in vitro or ex vivo.

In an embodiment of the present disclosure (including, for example, (1) to (23) above), CD201 is a protein known as a hematopoietic stem cell marker (for example, WO2017/205977 describes obtaining hematopoietic stem cells from human CB CD34⁺ cells using CD201 as a marker). CD201 includes a protein having the amino acid sequence of a protein represented by NCBI RefSeq Accession number NP_006395.2 or UniProt ID Q9UNN8. CD201 may also be referred to as cluster of differentiation 201 or EPCR.

In an embodiment of the present disclosure (including, for example, (1) to (23) above), CD90 is a protein known as a hematopoietic stem cell marker (for example, Rix et al., Front Physiol. 2022 Sep 30; 13:1009160 describes CD90 as a hematopoietic stem cell marker). CD90 includes a protein having the amino acid sequence of a protein represented by NCBI RefSeq Accession number NP_001298089.1 or UniProt ID P04216. CD90 may also be referred to as Cluster of Differentiation 90.

In an embodiment of the present disclosure (including, for example, (1) to (23) above), CD41 is a membrane protein mainly expressed on megakaryocytes and platelets. CD34⁺ and CD41⁺ cells are cells inclined toward differentiation into the megakaryocytic lineage.

In an embodiment of the present disclosure (including, for example, (1) to (23) above), it is preferable that the KDM5 degrader comprises at least one component (A) selected from the group consisting of a compound represented by general formula (I) and a salt thereof, and a compound represented by general formula (II) and a salt thereof.

In an embodiment of the present disclosure (including, for example, (1) to (23) above), it is preferable that the LSD1 inhibitor comprises at least one component (B) selected from the group consisting of tranylcypromine and a salt thereof.

In an embodiment of the present disclosure (including, for example, (1) to (23) above), tranylcypromine includes a compound with CAS registry number 155-09-9 or PubChem CID 96025309. Tranylcypromine is also sometimes called 2-PCPA. Tranylcypromine includes (1R,2S)-2-phenylcyclopropan-1-amine. In this specification, tranylcypromine may be referred to as 2-PCPA for abbreviation, but they can be used interchangeably.

In an embodiment of the present disclosure (including, for example, (1) to (23) above), the PI3K activator includes an activator of phosphatidylinositol 3-kinase. The PI3K activator includes an agonist of PI3K, and for example, includes 740Y-P or a salt thereof. 740Y-P includes a compound with CAS registry number 1236188-16-1 or PubChem CID 90488730.

In an embodiment of the present disclosure (including, for example, (1) to (23) above), culturing includes incubating cells under conditions suitable for growth or maintenance. Culturing may include a step of generating a cell-containing composition by contacting a medium and cells, and a step of incubating the cell-containing composition. The incubating step may be performed with the cell-containing composition standing still or with stirring. Incubation may be performed at about 37°C and in an atmosphere of about 5% CO₂. Contacting includes, for example, seeding cells in a medium or mixing cells and a medium. Culturing may be performed in an albumin-free or serum-free medium. Free refers to a state in which the target component is not added to the medium, a state in which the medium does not contain the target component at all, or a state in which the medium does not contain the component at a concentration above the detection limit. Albumin-free includes substantially albumin-free.

In an embodiment of the present disclosure (including, for example, (1) to (23) above), culturing may be performed in the presence of a cell adhesion factor (e.g., fibronectin). Culturing may be performed under conditions where hematopoietic stem cells and a cell adhesion factor can come into contact. Culturing may be performed, for example, with the inner surface (e.g., bottom) of a culture vessel coated with a cell adhesion factor.

In an embodiment of the present disclosure (including, for example, (1) to (23) above), culturing may include expansion of hematopoietic stem cells under conditions sufficient for the maintenance or proliferation of hematopoietic stem cells. Proliferation may include, for example, expansion of hematopoietic stem cells 10-fold or more from the start of culture. This fold increase may be 10, 50, 100, 200, 300, 400, or 500-fold or more, and may be within a range of any two of those values.

In an embodiment of the present disclosure (including, for example, (1) to (23) above), the cells used for culturing may be umbilical cord blood (CB) cells, peripheral blood cells, bone marrow cells, or a cell population thereof. The cells include cells collected from CB, cells collected from peripheral blood, and cells collected from bone marrow. These cells or cell populations may be CD34⁺ cells. The cells may be, for example, CB CD34⁺ cells (CD34⁺ cells derived from CB), peripheral blood CD34⁺ cells (CD34⁺ cells derived from peripheral blood), or bone marrow CD34⁺ cells (CD34⁺ cells derived from bone marrow). These cells may be purchased from a manufacturing or sales company (e.g., StemExpress or Lonza), or may be obtained by sorting CD34⁺ cells from CB, peripheral blood, or bone marrow. In another embodiment of the present disclosure, the cells used for culturing or obtained from culturing include stem cells, hematopoietic stem cells, or CD34⁺, CD201⁺, CD90⁺ or CD41⁺ cells. The cells or cell population thereof used for culturing may also be referred to as starting cells or a starting cell population. In an embodiment of the present disclosure (including, for example, (1) to (23) above), the cells used for culturing or obtained from culturing may be mammalian cells. Mammals include, for example, humans, monkeys, animals of the order Rodentia (mice, hamsters, etc.), rabbits, dogs, cats, horses, cattle, sheep, pigs, goats, marmosets, etc. In an embodiment of the present disclosure (including, for example, (1) to (23) above), the cells may exist in a state of a cell population.

In an embodiment of the present disclosure (including, for example, (1) to (23) above), a cell population includes, for example, a plurality of cells generated by cell division. In an embodiment of the present disclosure, the proportion of CD201⁺, CD90⁺ or CD41⁺ cells to the total cells in the medium after culturing may be, for example, 5% or more. This proportion may be, for example, 2, 3, 4, 5, 8, 10, 15, 20, 25, 30, 35, 40, 45, or 50% or more, and may be within a range of any two of those values. This proportion may be, for example, 5 to 50%, 15 to 40%, or 25 to 40%. The proportion of CD201⁺ cells is preferably 10% or more, more preferably 15% or more, and still more preferably 25% or more in order to efficiently obtain hematopoietic stem cells. The proportion of CD90⁺ cells is preferably 13% or more, and more preferably 15% or more in order to efficiently obtain hematopoietic stem cells. In an embodiment of the present disclosure, the proportion of CD201⁺ or CD90⁺ cells in the cell population after culturing may be 5-fold or more compared to before culturing. This fold increase may be, for example, 10, 20, 50, 100, or 200-fold or more, and may be within a range of any two of those values. The proportion of CD41⁺ cells is preferably 13% or more, and more preferably 15% or more for differentiation into megakaryocytes. The cell proportion may be the proportion after culturing for the culture time described below.

In an embodiment of the present disclosure (including, for example, (1) to (23) above), hematopoietic stem cells include stem cells that can differentiate into blood lineage cells. Hematopoietic stem cells can be collected from the umbilical cord, bone marrow, placenta, and peripheral blood. Human hematopoietic stem cells include CD34⁺ cells. Human hematopoietic stem cells may be CD34⁺CD38⁻. Human hematopoietic stem cells may be CD34⁺CD201⁺CD90⁺, CD34⁺CD201⁺ or CD34⁺CD41⁺. Human hematopoietic stem cell markers include CD34⁺, CD201⁺, CD90⁺ or CD41⁺. Hematopoietic stem cells include long-term hematopoietic stem cells.

In an embodiment of the present disclosure (including, for example, (1) to (23) above), the cell culture time may be, for example, 0.5, 1, 2, 3, 4, 5, 6, 8, 10, 12, 14, 16, 17, 18, 20, 30, or 35 days or more, and may be within a range of any two of those values. This culture time may be, for example, 1 to 20 days, 3 to 17 days, 4 to 16, or 5 to 14 days. Culture includes the time for incubating the medium containing cells.

In an embodiment of the present disclosure (including, for example, (1) to (23) above), the seeding density of cells during culturing may be 1000 cells/mL. This value may be 1000, 3000, 5000, 10000, 30000, 50000, 70000, 100000 or 150000 or more, and may be within a range of any two of those values.

In an embodiment of the present disclosure (including, for example, (1) to (23) above), the composition or medium includes a medium used for cell culture. The base component of the medium may be any general base component, and its composition is not limited. The base component of the medium may include, for example, amino acids, peptides, proteins, inorganic salts, vitamins, minerals, or a carbon source (e.g., glucose). The base component of the medium may include, for example, GlutaMAX, L-alanyl-L-glutamine dipeptide, L-glutamine, ITS-X, insulin, transferrin (apo), sodium selenite, ethanolamine, or Flt3 ligand. The medium may include, for example, a basal medium for cell culture. Basal media include, for example, S-clone SF-3 medium, F12 medium, StemSpan (Stem Cell technologies), STEMα (STEM ALPHA), StemPro-34 serum-free medium (Gibco, Invitrogen), StemPro MSC serum-free medium (Invitorogen), HSC-CFU medium (Miltenyl Biotech), S-Clone serum-free medium (SF-02, SF-03, CM-B, SF-B) (Sanko Junyaku), HPGM medium (Sanko Junyaku), AIM V medium (Invitrogen), Marrow MAX bone marrow medium (Invitrogen), KnockOut DMEM/F-12 medium (Invtrogen), Stemline hematopoietic stem cell expansion medium (Sigma), SYN serum-free medium (SYN H, SYN B) (AbCys SA), SPE IV medium (AbCys SA), MyeloCult medium (StemCell Technologies), HPG serum-free medium (Lonza), UltraCULTURE medium (Lonza), Opti-MEM medium (Gibco, Invitrogen etc.), MEM medium (Gibco, Invitrogen etc.), MEMα (Gibco, Invitrogen etc.), DMEM medium (Gibco, Invitrogen etc.), IMDM medium (Wako Pure Chemical etc.), PRMI1640 medium (Gibco, Invitrogen etc.), Ham F-12 medium (Gibco etc.), RD medium, and the like. The medium is preferably an albumin-free (e.g., GenBank accession number AAN17825.1) or serum-free medium. In this case, problems such as inhibition of stable undifferentiation and biological contamination can be suppressed. The medium may include Soluplus^{®} (CAS registry number 402932-23-4). Soluplus is mainly composed of a graft polymer of N-vinylcaprolactam/vinyl acetate copolymer and oxirane. The medium may include an antibiotic (e.g., penicillin or streptomycin). The medium may include hematopoietic stem cells, CD201⁺ cells, CD90⁺ cells, CD41⁺ cells, or CB cells. The medium may be a medium for culturing these cells. The medium may be a liquid medium or a solid medium. Culturing cells using a medium includes culturing the cells in the medium.

In an embodiment of the present disclosure (including, for example, (1) to (23) above), the composition or medium may further comprise CB CD34⁺ cells. This composition or medium includes a composition or medium that is enriched with CD201⁺, CD90⁺ or CD41⁺ cells upon incubation.

In an embodiment of the present disclosure (including, for example, (1) to (23) above), the concentration of the PI3K activator in the composition or medium may be, for example, 1, 2, 3, 4, 5, 6, 7, 8, 9, 10, 15, 20, 30, or 50 µmol/L or more, and may be within a range of any two of those values. This concentration may be, for example, 1 to 25 µmol/L, 2 to 20 µmol/L, or 3 to 10 µmol/L. This concentration is preferably 2 µmol/L or more, more preferably 3 µmol/L or more, and still more preferably 4 µmol/L or more, in order to efficiently obtain hematopoietic stem cells.

In an embodiment of the present disclosure (including, for example, (1) to (23) above), the concentration of the TPO receptor agonist in the composition or medium may be, for example, 0.01, 0.03, 0.05, 0.08, 0.1, 0.12, 0.15, 0.2, 0.3, 0.5, 1, or 2 µmol/L or more, and may be within a range of any two of those values. This concentration may be, for example, 0.01 to 0.3 µmol/L, 0.05 to 0.2 µmol/L, or 0.08 to 0.15 µmol/L. This concentration is preferably 0.03 µmol/L or more, more preferably 0.05 µmol/L or more, and still more preferably 0.08 µmol/L or more, in order to efficiently obtain hematopoietic stem cells.

In an embodiment of the present disclosure (including, for example, (1) to (23) above), the concentration of UM729 or a salt thereof in the composition or medium may be, for example, 0.1, 0.3, 0.5, 0.8, 1, 1.3, 1.5, 1.8, 2, 3, 4, 5, 6, 7, 8, 9, 10, 20, 30, or 30 µmol/L or more, and may be within a range of any two of those values. This concentration may be, for example, 0.1 to 3 µmol/L, 0.5 to 2 µmol/L, or 0.8 to 1.5 µmol/L. This concentration is preferably 0.3 µmol/L or more, more preferably 0.5 µmol/L or more, and still more preferably 0.8 µmol/L or more, in order to efficiently obtain hematopoietic stem cells.

In an embodiment of the present disclosure (including, for example, (1) to (23) above), the concentration of 2-PCPA or a salt thereof in the composition or medium may be, for example, 1, 3, 5, 8, 10, 13, 15, 18, 20, 30, 40, or 50 µmol/L or more, and may be within a range of any two of those values. This concentration may be, for example, 1 to 30 µmol/L, 5 to 20 µmol/L, or 8 to 15 µmol/L. This concentration is preferably 3 µmol/L or more, more preferably 5 µmol/L or more, and still more preferably 8 µmol/L or more, in order to efficiently obtain hematopoietic stem cells. The 2-PCPA or a salt thereof may be, for example, tranylcypromine hydrochloride.

In an embodiment of the present disclosure (including, for example, (1) to (23) above), the composition or medium may contain another protein component at a concentration of 1 ng/mL or more. This concentration may be, for example, 1, 5, 8, 10, 15, 20, 30, or 50 ng/mL or more, and may be within a range of any two of those values. This concentration may be, for example, 1 to 30 µmol/L, 5 to 20 µmol/L, or 8 to 12 µmol/L. The other protein component may be, for example, Flt3 ligand.

In an embodiment of the present disclosure (including, for example, (1) to (23) above), the composition or medium may contain a modified polyalkylene glycol as another additive component. The concentration of the modified polyalkylene glycol may be, for example, 0.001, 0.005, 0.008, 0.01, 0.012, 0.015, 0.02, 0.03, 0.05, 0.1, 0.2, 0.5, 1, 2, or 5% (w/v) or more, and may be within a range of any two of those values. This concentration may be, for example, 0.001 to 0.1% (w/v), 0.005 to 0.05% (w/v), 0.005 to 0.02% (w/v), 0.008 to 0.02% (w/v) or 0.008 to 0.015% (w/v). The modified polyalkylene glycol may be, for example, a polyalkylene glycol modified by a copolymer of a polyvinylcaprolactam block and a polyvinyl acetate block. The polyalkylene glycol modified by a copolymer of a polyvinylcaprolactam block and a polyvinyl acetate block may be, for example, a polyethylene glycol modified by a copolymer of a polyvinylcaprolactam block and a polyvinyl acetate block. The modified polyalkylene glycol may be, for example, a polyvinylcaprolactam-polyvinyl acetate-polyethylene glycol graft copolymer. The modified polyalkylene glycol may be, for example, a graft copolymer obtained from (i) N-vinylcaprolactam, (ii) vinyl acetate, and (iii) polyether. The polyether may be polyethylene glycol. The modified polyalkylene glycol may be, for example, a compound obtained by free-radical polymerization of a mixture of (i) to (iii) above. For example, (i) may be 40 to 60 wt%, (ii) may be 15 to 35 wt%, and (iii) may be 10 to 30 wt%. (i) may be, for example, 40, 45, 50, 55, 57 or 60 wt%, and may be within a range of any two of those values. (ii) may be, for example, 15, 20, 25, 30, or 35 wt%, and may be within a range of any two of those values. (iii) may be, for example, 10, 13, 15, 20, 25, or 30 wt%, and may be within a range of any two of those values. The above (i) to (iii) may be 100 wt% in total. The modified polyalkylene glycol may be, for example, a compound represented by the following structural formula (S) or a salt thereof.

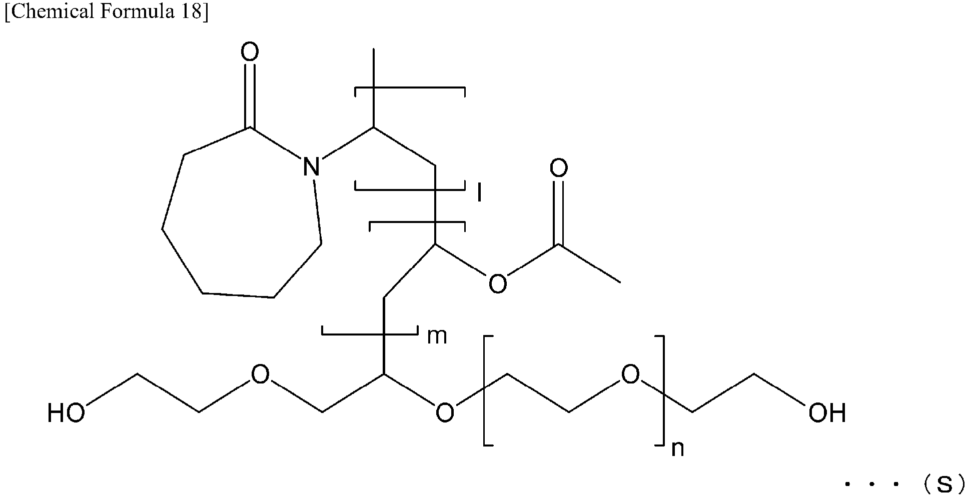

Here, the wt% of l, m, and n may be, for example, 40 to 60 wt%, 15 to 35 wt%, and 10 to 30 wt%, respectively. The wt% of l may be, for example, 40, 45, 50, 55, 57 or 60, and may be within a range of any two of those values. The wt% of m may be, for example, 15, 20, 25, 30, or 35, and may be within a range of any two of those values. The wt% of n may be, for example, 10, 13, 15, 20, 25, or 30, and may be within a range of any two of those values. The wt% of l, m, and n above may be 100 wt% in total. The wt% of l, m, and n may be, for example, about 57, about 30, and about 13 wt%, respectively. The degrees of polymerization of l, m, and n may be, for example, 60 to 160, 470 to 1110, and 480 to 1130, respectively. The degree of polymerization of l may be, for example, 60, 70, 80, 90, 100, 110, 120, 130, 140, 150 or 160, and may be within a range of any two of those values. The degree of polymerization of m may be, for example, 470, 500, 550, 600, 650, 700, 750, 780, 800, 850, 900, 950, 1000, 1050 or 1110, and may be within a range of any two of those values. The degree of polymerization of n may be, for example, 480, 500, 550, 600, 650, 700, 750, 800, 850, 900, 950, 1000, 1050 or 1130, and may be within a range of any two of those values. The degree of polymerization may be the average degree of polymerization. The weight-average molecular weight (Mw) of the modified polyalkylene glycol may be, for example, 70,000 g/mol or more. This value may be, for example, 70,000, 80,000, 90,000, 100,000, 120,000, 140,000, 150,000, 160,000, or 170,000 g/mol, and may be within a range of any two of those values. This value may be, for example, 70,000 to 170,000 g/mol, 80,000 to 160,000 g/mol, 90,000 to 140,000 g/mol, 100,000 to 130,000 g/mol, or 110,000 to 125,000 g/mol. This value may be about 118,000 g/mol. The modified polyalkylene glycol may be, for example, Soluplus. Soluplus is a compound having the structure of the above formula (S). The l, m, and n of Soluplus may have the above wt% or degree of polymerization. The production method of the modified polyalkylene glycol may employ, for example, the method described in US 2008/0293828 A1, US 2010/0204425 A1, or US 2018/0305636 A1. The components described in these documents may be used as the components of (i) to (iii) above.

In an embodiment of the present disclosure (including, for example, (1) to (23) above), the container includes a culture vessel or a medical container. When used for culturing, a culture vessel may be used, and when used for hematopoietic stem cell transplantation, a medical container may be used. The culture vessel may be, for example, a plate type, a petri dish type, a flask type, or a bottle type. The medical container may be, for example, a bottle type, a bag type, or a syringe type.

In an embodiment of the present disclosure (including, for example, (1) to (23) above), the collecting step may include, for example, transferring desired cells or a cell population from a container containing the cells or cell population to another container. The collection may include a step of sorting or purifying. The collecting step may be performed in a sterile environment.

In an embodiment of the present disclosure (including, for example, (1) to (23) above), the composition includes, for example, a medium or an additive. The medium may be, for example, a medium for culturing hematopoietic stem cells or CB CD34⁺ cells. The additive may be an additive for supplying additional nutrients to a basal medium.

In an embodiment of the present disclosure (including, for example, (1) to (23) above), the pharmaceutical composition may be produced by mixing cells (e.g., hematopoietic stem cells) with one or more pharmaceutically acceptable carriers and by any method known in the field of pharmaceutics. The pharmaceutical composition may include a stabilizer, a buffer, or a pH adjuster. The dosage, administration interval, administration method, and administration route are not particularly limited and can be appropriately selected depending on the patient's age, weight, symptoms, target organ, and the like. It is preferable that the pharmaceutical composition contains hematopoietic stem cells in a therapeutically effective amount or an effective amount that exerts a desired action. In one embodiment of the present disclosure, a therapeutically effective amount includes the amount necessary for clinical observation of improvement or suppression of symptoms in a patient. In one embodiment of the present disclosure, pharmaceutically acceptable includes a state suitable for use within the scope of sound medical judgment, commensurate with a reasonable benefit/risk ratio.

In an embodiment of the present disclosure (including, for example, (1) to (23) above), a subject (including a patient) includes a human or a non-human mammal (e.g., one or more of mice, guinea pigs, hamsters, rats, mice, rabbits, pigs, sheep, goats, cattle, horses, cats, dogs, marmosets, monkeys, or chimpanzees). A patient may be a patient diagnosed with a disease (e.g., cancer or immunodeficiency) or a patient in need of treatment for a disease.

In an embodiment of the present disclosure (including, for example, (1) to (23) above), being enriched includes an increase in the proportion of a specific type of cell in a medium or cell population. A cultured fraction enriched with CD201⁺, CD90⁺ or CD41⁺ cells may have a quantitative proportion of the number of CD201⁺, CD90⁺ or CD41⁺ cells to the total number of cells in the medium of, for example, 5% or more. This proportion may be, for example, 2, 3, 4, 5, 8, 10, 15, 20, 25, 30, 35, 40, 45, or 50% or more, and may be within a range of any two of those values. This proportion may be, for example, 8 to 50%, 15 to 40%, or 25 to 40%. The proportion of CD201⁺ cells is preferably 10% or more, more preferably 15% or more, and still more preferably 25% or more in order to efficiently obtain hematopoietic stem cells. The proportion of CD90⁺ cells is preferably 13% or more, and more preferably 15% or more in order to efficiently obtain hematopoietic stem cells. A cultured fraction includes a cell-containing composition obtained by culturing cells. The proportion of CD41⁺ cells is preferably 10% or more, and more preferably 15% or more. A cultured fraction includes a fraction before sorting or purification treatment is performed after culturing the cells.

In an embodiment of the present disclosure (including, for example, (1) to (23) above), a salt is not particularly limited and includes, for example, inorganic salts or organic salts (see, for example, "Bharate et al., Drug Discov Today. 2021 Feb; 26(2): 384-398." or "Berge et al., J Pharm Sci. 1977 Jan; 66(1): 1-19."). A salt includes, for example, metal salts, ammonium salts, salts with organic bases, salts with inorganic acids, salts with organic acids, salts with basic or acidic amino acids, and the like. Metal salts include, for example, alkali metal salts (sodium salts, potassium salts, etc.), alkaline earth metal salts (calcium salts, magnesium salts, barium salts, etc.), aluminum salts, and the like. Salts with organic bases include, for example, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, and the like. Salts with inorganic acids include, for example, salts with hydrochloric acid, hydrobromic acid, nitric acid, sulfuric acid, phosphoric acid, and the like. Salts with organic acids include, for example, salts with formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, p-toluenesulfonic acid, and the like. Salts with basic amino acids include, for example, salts with arginine, lysine, ornithine, and the like. Salts with acidic amino acids include, for example, salts with aspartic acid, glutamic acid, and the like. A salt includes a pharmaceutically acceptable salt. In one embodiment of the present disclosure, pharmaceutically acceptable includes a form having a reasonable benefit for pharmaceutical use.

In an embodiment of the present disclosure (including, for example, (1) to (23) above), ⁺ includes being recognized as positive for the molecule specified by the term immediately preceding it.

Any publication cited herein is incorporated by reference in its entirety. In this specification, "or" is used when "at least one or more" of the items listed in the text can be adopted. The same applies to "or". For example, CD201⁺ or CD90⁺ includes any of CD201⁺, CD90⁺, and CD201⁺ and CD90⁺. In this specification, when "within a range of two values" is specified, the range includes the two values themselves. In this specification, "A to B" includes A and B. In this specification, "(1) to (23) above" includes a reference to any one or more of (1), (2), (3), (4), (5), (6), (7), (8), (9), (10), (11), (12), (13), (14), (15), (16), (17), (18), (19), (20), (21), (22), or (23).

Furthermore, a compound represented by the following formula (III) or (IV) may be used as the KDM5 degrader which is component (A) in (1) above.

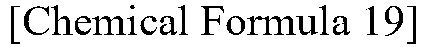

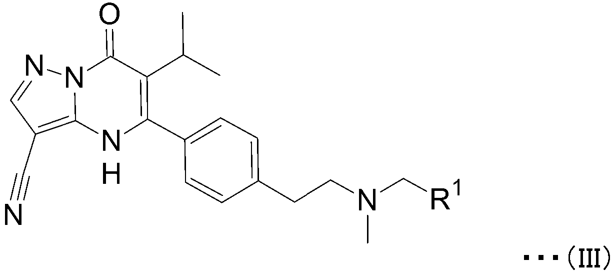

In formula (III), R¹ is a linear alkyl group having 1 to 6 carbon atoms.

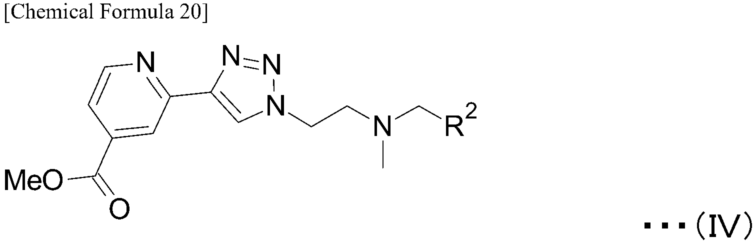

In formula (IV), R² is a linear alkyl group having 1 to 6 carbon atoms.

As the compound represented by said formula (III), a compound represented by the following formula (NPC-4034) is preferable.

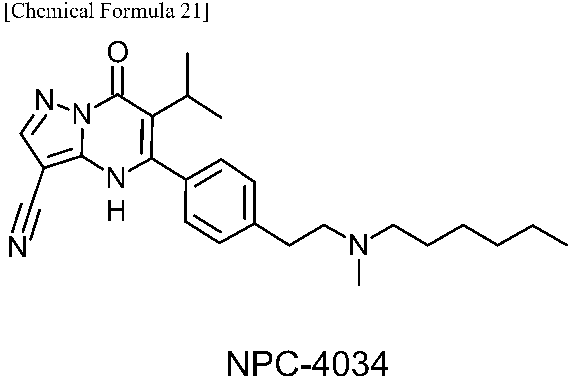

As the compound represented by said formula (IV), a compound represented by the following formula (T-31) is preferable.

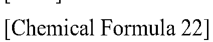

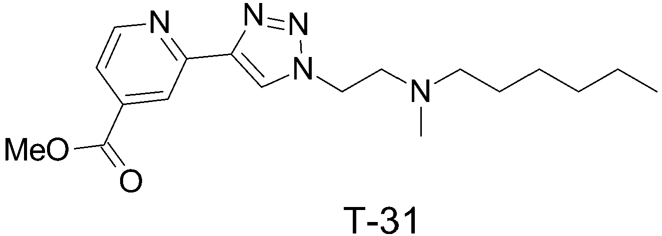

These compounds can be used under the same conditions as the compounds represented by formulas (I) and (II).

Although the embodiments of the present disclosure have been described above, these are examples of forms that can be included in the present disclosure, and the present disclosure is not limited to these, and various other configurations can be adopted. The present disclosure can also adopt each configuration or feature described in the above embodiments in combination or independently.

### <Actions and Effects>

According to the present disclosure, a composition and a medium used for cell culture can be provided. Specifically, a cell population comprising CD34⁺ cells, CD201⁺ cells, CD90⁺, and CD41⁺ cells can be proliferated.

### Examples

Hereinafter, the present disclosure will be further described by way of examples, but the present disclosure is not limited to these.

In the examples, compound 126 (TIA01-126) is a compound represented by formula (I), wherein in formula (I), A is an m-phenylene group, L is an n-pentylene group (a linear alkylene group having 5 carbon atoms), and X is a group represented by -(CH₂)ₜO-, and t is 6. Compound 135 (TIA02-135) is a compound represented by formula (II), wherein in formula (II), R is a methyl group, and Z is an n-nonylene group (a linear alkylene group having 9 carbon atoms).

### <Example 1>

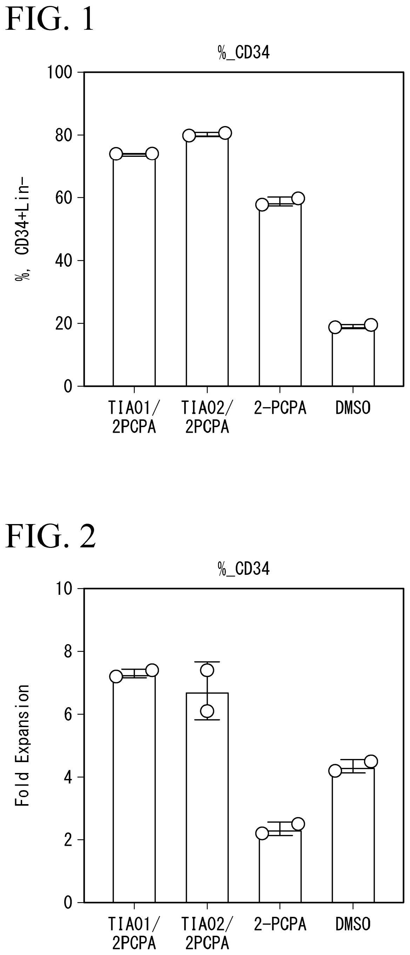

Figure 1 shows the results of analyzing human umbilical cord blood CD34⁺ cells cultured with compound(s) in a Soluplus-based medium [IMDM (Life Technologies) supplemented with 0.01% Soluplus (BASF), 1% Insulin-Transferrin-Selenium-Ethanolamine (ITSX; Life Technologies), 1% Penicillin-Streptomycin (P/S; Life Technologies), 0.5 µmol/L 740Y-P, 0.1 µmol/L Butyzamide, and 10 ng/mL recombinant FMS like-Tyrosine kinase 3 Ligand (FLT3L; PeproTech)] for 10 days using Flow Cytometry. The vertical axis shows the percentage of CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 2>

Figure 2 shows the results of analyzing human umbilical cord blood CD34⁺ cells cultured with compound(s) in a Soluplus-based medium for 10 days using Flow Cytometry. The vertical axis shows the expansion rate of CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 3>

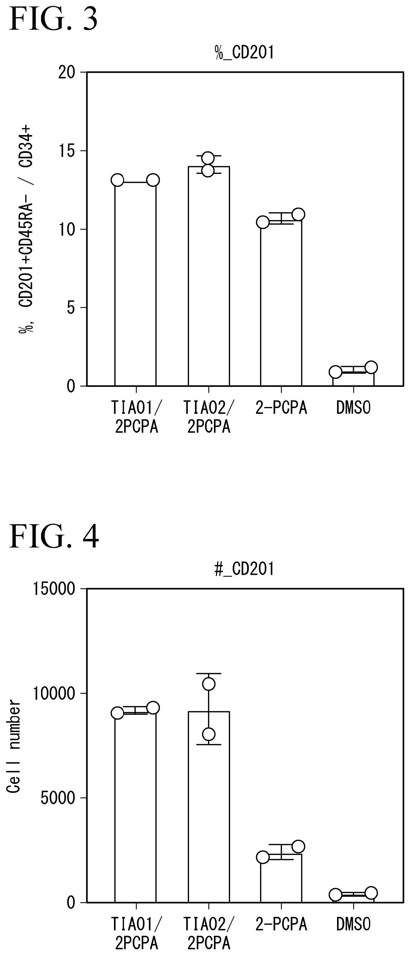

Figure 3 shows the results of analyzing human umbilical cord blood CD34⁺ cells cultured with compound(s) in a Soluplus-based medium for 10 days using Flow Cytometry. The vertical axis shows the percentage of CD201⁺CD45RA⁻ cells in CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 4>

Figure 4 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD201⁺CD45RA⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 5>

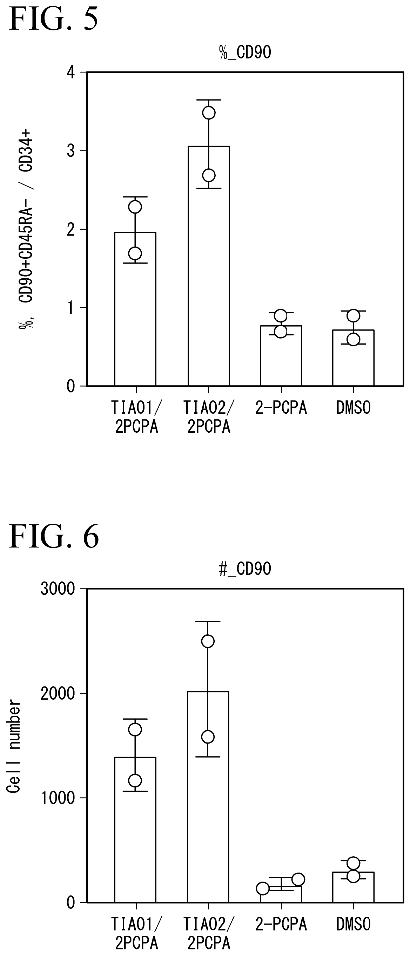

Figure 5 shows the results of analyzing human umbilical cord blood CD34⁺ cells cultured with compound(s) in a Soluplus-based medium for 10 days using Flow Cytometry. The vertical axis shows the percentage of CD90⁺CD45RA⁻ cells in CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 6>

Figure 6 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD90⁺CD45RA⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; 2PCPA: 5.0 µmol/L 2PCPA; and DMSO: 0.1% DMSO.

### <Example 7>

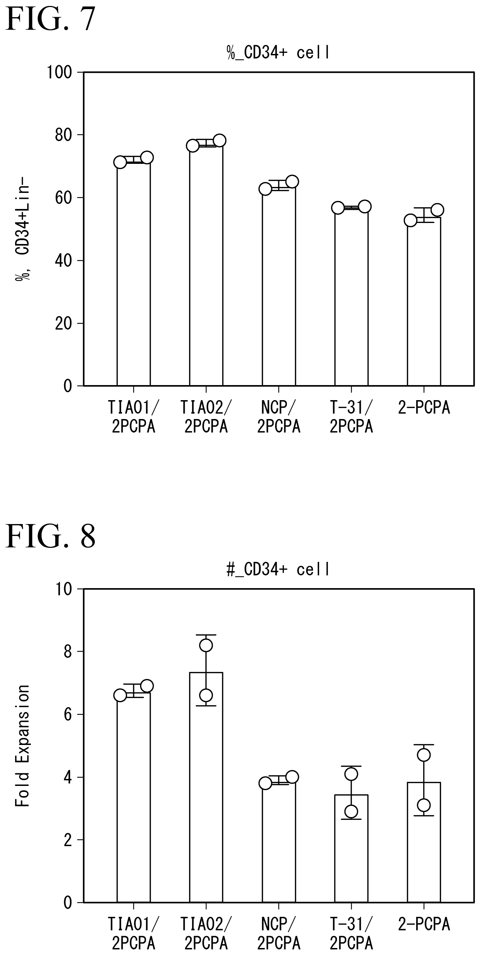

Figure 7 shows the results of analyzing human umbilical cord blood CD34⁺ cells cultured with compound(s) in a Soluplus-based medium for 10 days using Flow Cytometry. The vertical axis shows the percentage of CD34⁺Lineage marker⁻ cells. The horizontal axis shows the compound(s), which are, respectively, TIA01/2PCPA: 5.0 µmol/L TIA01-126 and 5.0 µmol/L 2-PCPA; TIA02/2PCPA: combination of 1.0 µmol/L TIA02-135 and 5.0 µmol/L 2-PCPA; NCP/2PCPA: combination of 1.0 µmol/L NCP-4034 and 5.0 µmol/L 2-PCPA; T-31/2PCPA: combination of 1.0 µmol/L T-31 and 5.0 µmol/L 2-PCPA; and 2PCPA: 5.0 µmol/L 2PCPA.

### <Example 8>

Figure 8 shows the results of analyzing human umbilical cord blood CD34⁺ cells cultured with compound(s) in a Soluplus-based medium for 10 days using Flow Cytometry. The vertical axis shows the expansion rate of CD34⁺Lineage marker⁻ cells. The horizontal axis shows the compound(s), which are, respectively, TIA01/2PCPA: 5.0 µmol/L TIA01-126 and 5.0 µmol/L 2-PCPA; TIA02/2PCPA: 1.0 µmol/L TIA02-135 and 5.0 µmol/L 2-PCPA; NCP/2PCPA: combination of 1.0 µmol/L NCP-4034 and 5.0 µmol/L 2-PCPA; T-31/2PCPA: combination of 1.0 µmol/L T-31 and 5.0 µmol/L 2-PCPA; and 2PCPA: 5.0 µmol/L 2PCPA.

### <Example 9>

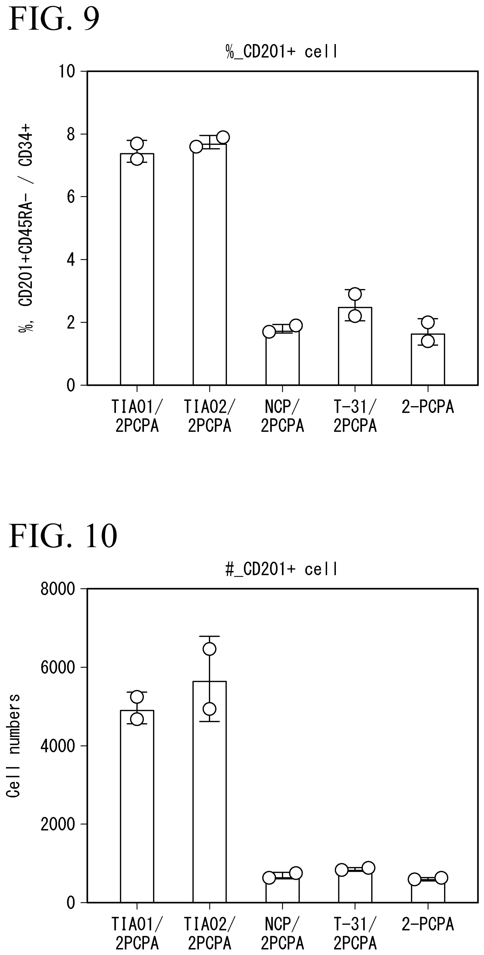

Figure 9 shows the results of analyzing human umbilical cord blood CD34⁺ cells cultured with compound(s) in a Soluplus-based medium for 10 days using Flow Cytometry. The vertical axis shows the percentage of CD201⁺CD45RA⁻ cells in CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; NCP/2PCPA: 1.0 µmol/L NCP-4034 and 5.0 µmol/L 2-PCPA; T-31/2PCPA: combination of 1.0 µmol/L T-31 and 5.0 µmol/L 2-PCPA; and 2PCPA: 5.0 µmol/L 2PCPA.

### <Example 10>

Figure 10 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD201⁺CD45RA⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: combination of 1.0 µmol/L TIA02-135 and 5.0 µmol/L 2-PCPA; NCP/2PCPA: combination of 1.0 µmol/L NCP-4034 and 5.0 µmol/L 2-PCPA; T-31/2PCPA: combination of 1.0 µmol/L T-31 and 5.0 µmol/L 2-PCPA; and 2PCPA: 5.0 µmol/L 2PCPA.

### <Example 11>

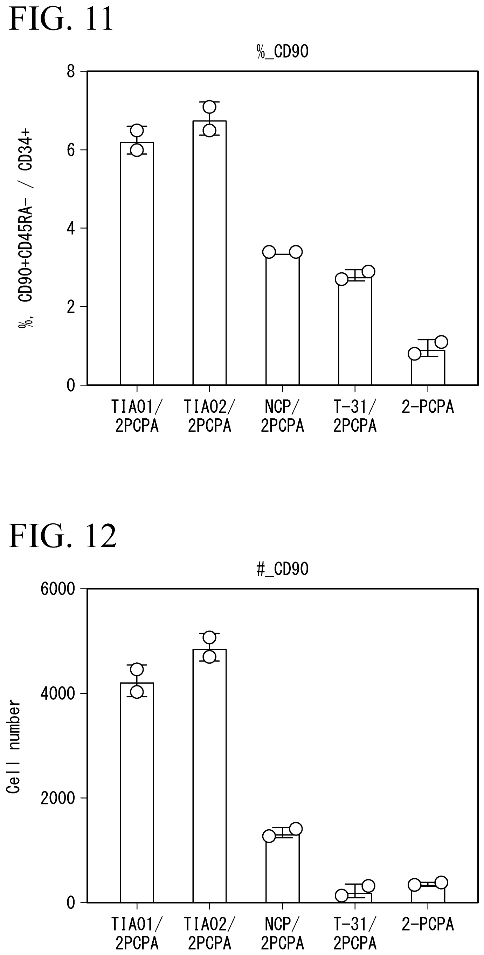

Figure 11 shows the results of analyzing human umbilical cord blood CD34⁺ cells cultured with compound(s) in a Soluplus-based medium for 10 days using Flow Cytometry. The vertical axis shows the percentage of CD90⁺CD45RA⁻ cells in CD34⁺Lineage marker⁻ cells. The horizontal axis shows the compound(s), which are, respectively, TIA01/2PCPA: combination of 5.0 µmol/L TIA01-126 and 5.0 µmol/L 2-PCPA; TIA02/2PCPA: 1.0 µmol/L TIA02-135 and 5.0 µmol/L 2-PCPA; NCP/2PCPA: 1.0 µmol/L NCP-4034 and 5.0 µmol/L 2-PCPA; T-31/2PCPA: 1.0 µmol/L T-31 and 5.0 µmol/L 2-PCPA; and 2PCPA: 5.0 µmol/L 2PCPA.

### <Example 12>

Figure 12 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD90⁺CD45RA⁻ cells. The horizontal axis shows the compound(s), which are, respectively, TIA01/2PCPA: 5.0 µmol/L TIA01-126 and 5.0 µmol/L 2-PCPA; TIA02/2PCPA: combination of 1.0 µmol/L TIA02-135 and 5.0 µmol/L 2-PCPA; NCP/2PCPA: 1.0 µmol/L NCP-4034 and 5.0 µmol/L 2-PCPA; T-31/2PCPA: combination of 1.0 µmol/L T-31 and 5.0 µmol/L 2-PCPA; and 2PCPA: 5.0 µmol/L 2PCPA.

### <Example 13>

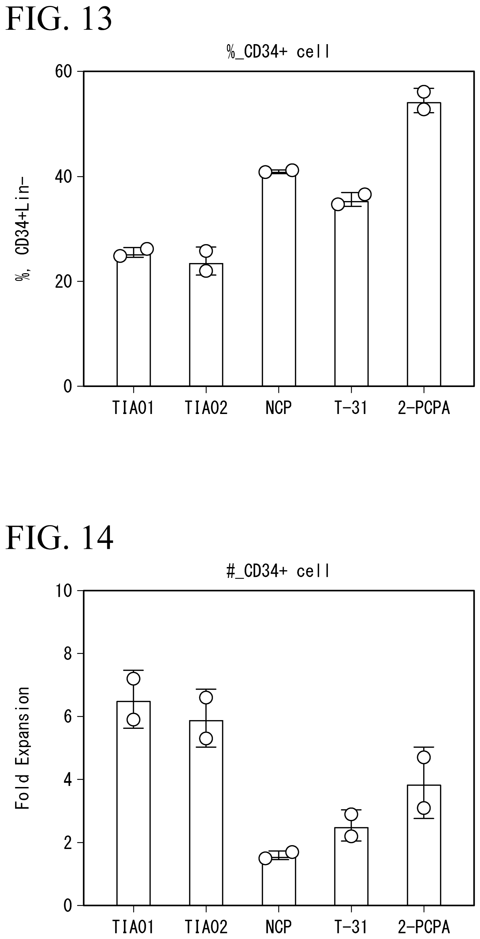

Figure 13 shows the results of analyzing human umbilical cord blood CD34⁺ cells cultured with compound(s) in a Soluplus-based medium for 10 days using Flow Cytometry. The vertical axis shows the percentage of CD34⁺Lineage marker⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 14>

Figure 14 shows the results of analyzing human umbilical cord blood CD34⁺ cells cultured with compound(s) in a Soluplus-based medium for 10 days using Flow Cytometry. The vertical axis shows the expansion rate of CD34⁺Lineage marker⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 15>

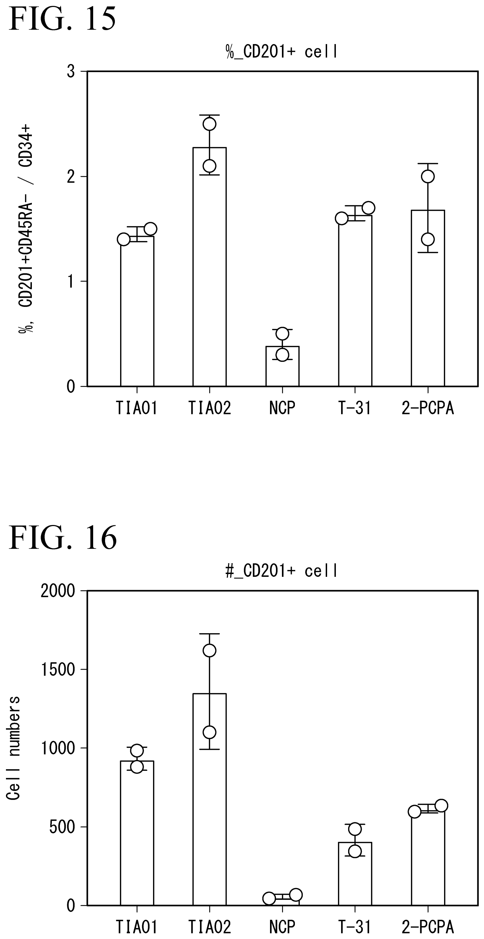

Figure 15 shows the results of analyzing human umbilical cord blood CD34⁺ cells cultured with compound(s) in a Soluplus-based medium for 10 days using Flow Cytometry. The vertical axis shows the percentage of CD201⁺CD45RA⁻ cells in CD34⁺Lineage marker⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 16>

Figure 16 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD201⁺CD45RA⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 17>

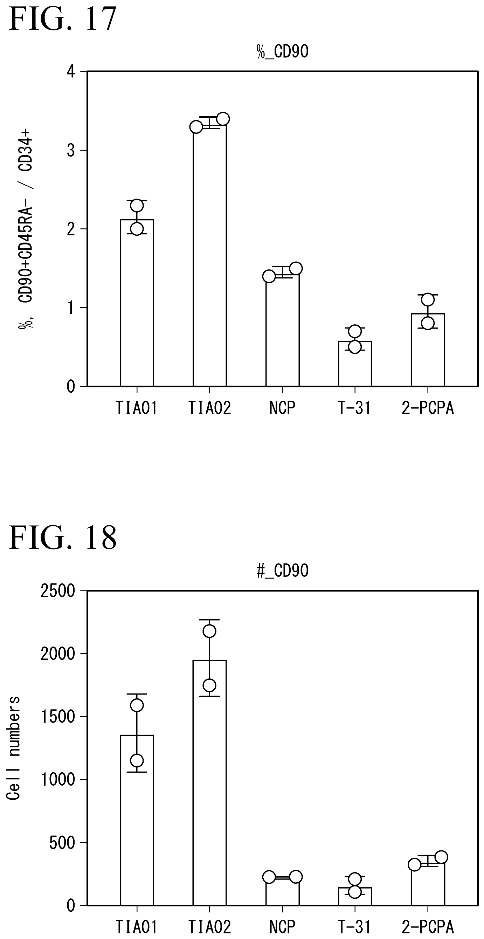

Figure 17 shows the results of analyzing human umbilical cord blood CD34⁺ cells cultured with compound(s) in a Soluplus-based medium for 10 days using Flow Cytometry. The vertical axis shows the percentage of CD90⁺CD45RA⁻ cells in CD34⁺Lineage marker⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 18>

Figure 18 shows the results of analyzing human umbilical cord blood CD34⁺ cells cultured with compound(s) in a Soluplus-based medium for 10 days using Flow Cytometry. The vertical axis shows the number of CD34⁺Lineage marker⁻CD90⁺CD45RA⁻ cells. The horizontal axis shows the compound(s), which are, respectively, TIA01: 5.0 µmol/L TIA01-126; TIA02: 1.0 µmol/L TIA02-135; NCP: 1.0 µmol/L NCP-4034; T-31: 1.0 µmol/L T-31; and 2PCPA: 5.0 µmol/L 2PCPA.

### <Example 19>

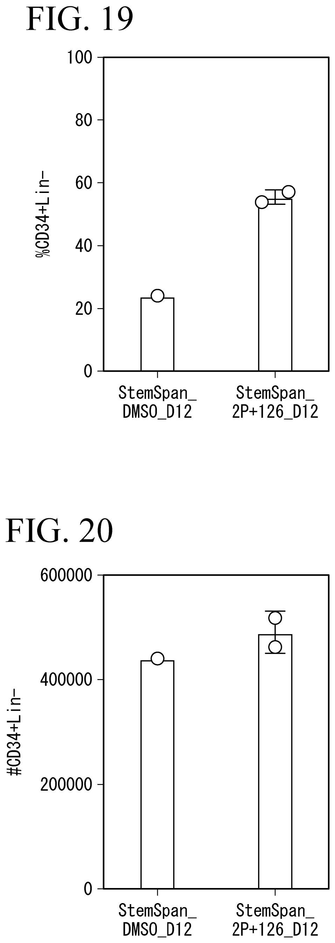

Figure 19 shows the results of analyzing human umbilical cord blood CD34⁺ cells cultured with compound(s) in an albumin-based medium [StemSpan SFEM (STEMCELL Technologies) supplemented with 100 ng/mL recombinant Stem cell Factor (SCF, PeproTech), 50 ng/mL recombinant TPO (PeproTech), 100 ng/mL FLT3L, 10 µg/mL Low Density Lipoprotein (LDL; STEMCELL Technologies)] for 12 days using Flow Cytometry. The vertical axis shows the percentage of CD34⁺Lineage marker⁻ cells. The horizontal axis shows the compound(s), which are, respectively, DMSO: 0.1% DMSO; and 2P+126: combination of 4.0 µmol/L TIA01-126 and 5.0 µmol/L 2-PCPA.

### <Example 20>

Figure 20 shows the results of analyzing human umbilical cord blood CD34⁺ cells cultured with compound(s) in an albumin-based medium for 12 days using Flow Cytometry. The vertical axis shows the number of CD34⁺Lineage marker⁻ cells. The horizontal axis shows the compound(s), which are, respectively, DMSO: 0.1% DMSO; and 2P+126: combination of 4.0 µmol/L TIA01-126 and 5.0 µmol/L 2-PCPA.

### <Example 21>

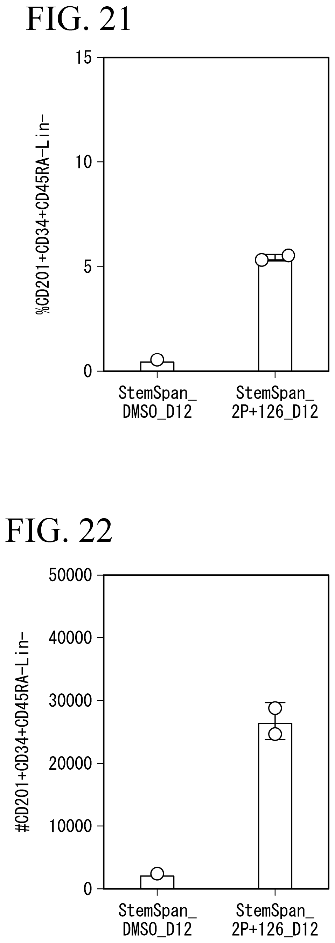

Figure 21 shows the results of analyzing human umbilical cord blood CD34⁺ cells cultured with compound(s) in an albumin-based medium for 12 days using Flow Cytometry. The vertical axis shows the percentage of CD201⁺CD45RA⁻ cells in CD34⁺Lineage marker⁻ cells. The horizontal axis shows the compound(s), which are, respectively, DMSO: 0.1% DMSO; and 2P+126: combination of 4.0 µmol/L TIA01-126 and 5.0 µmol/L 2-PCPA.

### <Example 22>

Figure 22 shows the results of analyzing human umbilical cord blood CD34⁺ cells cultured with compound(s) in an albumin-based medium for 12 days using Flow Cytometry. The vertical axis shows the number of CD34⁺Lineage marker⁻CD201⁺CD45RA⁻ cells. The horizontal axis shows the compound(s), which are, respectively, DMSO: 0.1% DMSO; and 2P+126: combination of 4.0 µmol/L TIA01-126 and 5.0 µmol/L 2-PCPA.

### <Example 23>

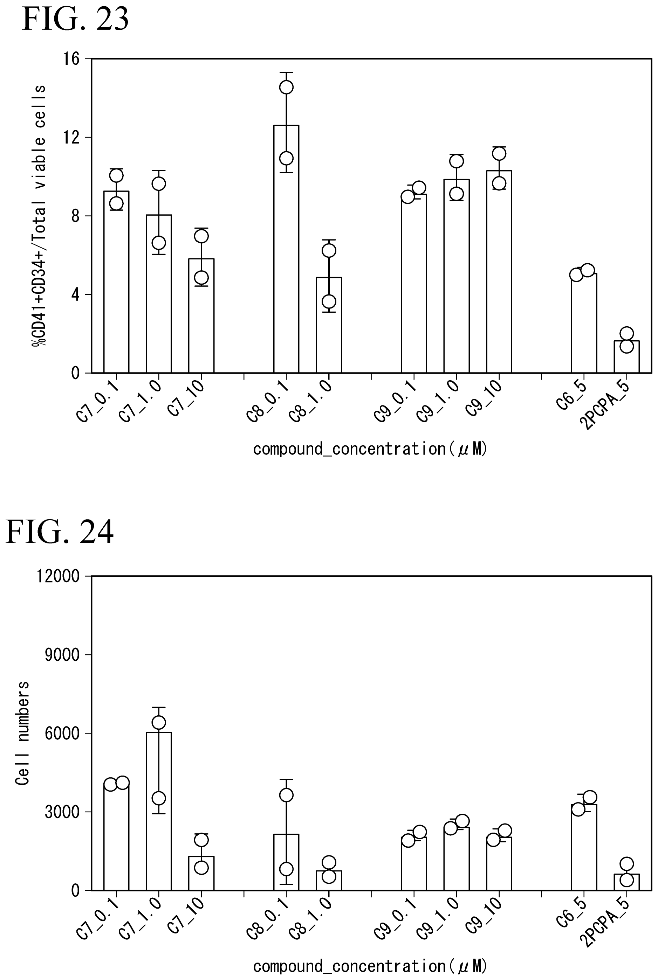

Figure 23 shows the results of analyzing human umbilical cord blood CD34⁺ cells cultured with compound(s) in a Soluplus-based medium for 10 days using Flow Cytometry. The vertical axis shows the percentage of CD41⁺CD34⁺ cells in all viable cells. The horizontal axis shows the compound(s) and their contents, which are, respectively, C7_0.1: 0.1 µmol/L TIA02-135; C7_1.0: 1.0 µmol/L TIA02-135; C7_10: 10 µmol/L TIA02-135; C8_0.1: 0.1 µmol/L NPC-4034; C8_1.0: 1.0 µmol/L NPC-4034; C9_0.1: 0.1 µmol/L T-31; C9_1.0: 1.0 µmol/L T31; C9_10: 10 µmol/L T31; C6_5: 5.0 µmol/L TIA01-126; and 2PCPA_5: 5.0 µmol/L 2-PCPA.

### <Example 24>

Figure 24 shows the results of analyzing human umbilical cord blood CD34⁺ cells cultured with compound(s) in a Soluplus-based medium for 10 days using Flow Cytometry. The vertical axis shows the number of CD41⁺CD34⁺ cells. The horizontal axis shows the compounds and their contents, which are the same as in Figure 23.

### <Example 25>

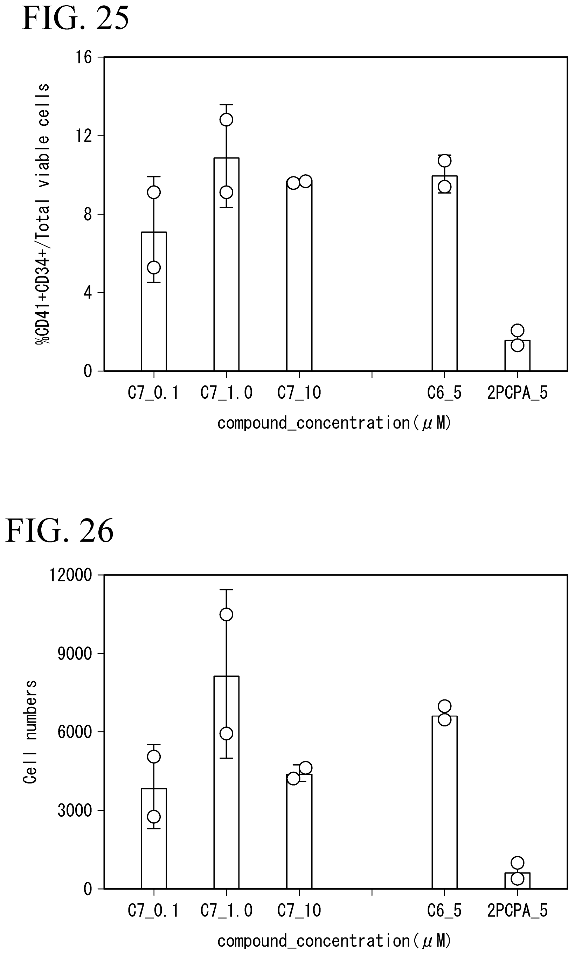

Figure 25 shows the results of analyzing human umbilical cord blood CD34⁺ cells cultured with compound(s) in a Soluplus-based medium for 10 days using Flow Cytometry. The vertical axis shows the percentage of CD41⁺CD34⁺ cells in all viable cells. The horizontal axis shows the compounds and their contents, which are the same types and contents of compounds as in Figure 23, each with the addition of 5.0 µmol/L of 2-PCPA in combination. 2PCPA_5: 5.0 µmol/L 2-PCPA.

### <Example 26>

Figure 26 shows the results of analyzing human umbilical cord blood CD34⁺ cells cultured with compound(s) in a Soluplus-based medium for 10 days using Flow Cytometry. The vertical axis shows the number of CD41⁺CD34⁺ cells. The horizontal axis shows the compounds and their contents, which are the same types and contents of compounds as in Figure 23, each with the addition of 5.0 µmol/L of 2-PCPA in combination, similar to Figure 25. 2PCPA_5: 5.0 µmol/L 2-PCPA.

### <Example 27>

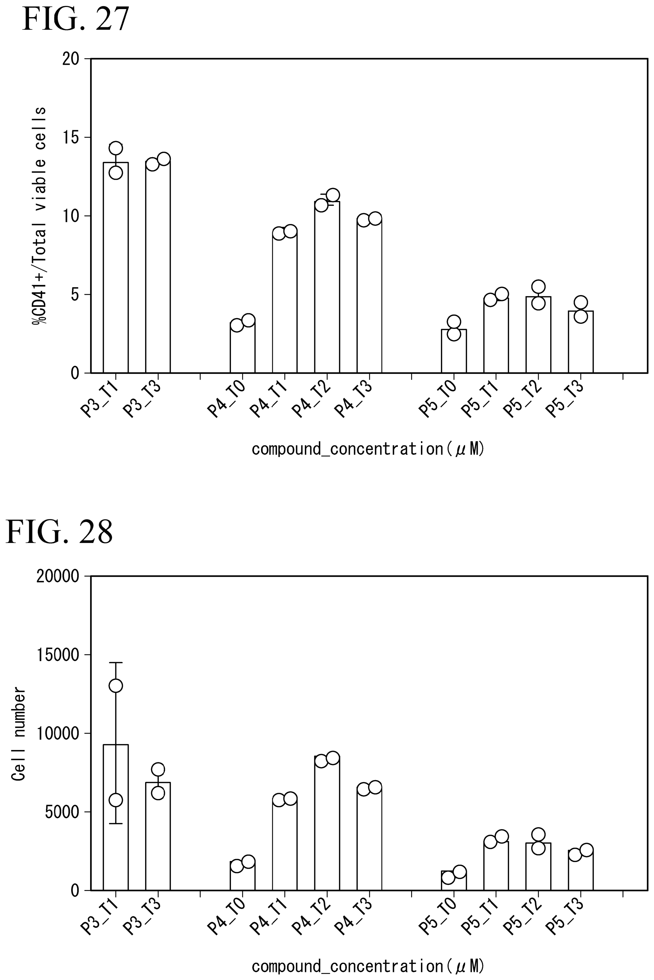

Figure 27 shows the results of analyzing human umbilical cord blood CD34⁺ cells cultured with compound(s) in a Soluplus-based medium for 10 days using Flow Cytometry. The vertical axis shows the percentage of CD41⁺CD34⁺ cells in all viable cells. The horizontal axis shows the compounds and their contents, which are, respectively, P3_T1: combination of 3 µmol/L 2-PCPA and 1.0 µmol/L T1A02-135; P3_T3: combination of 3 µmol/L 2-PCPA and 3 µmol/L T1A02-135; P4_T0: 4.0 µmol/L 2-PCPA; P4_T1: combination of 4.0 µmol/L 2-PCPA and 1.0 µmol/L T1A02-135; P4_T2: combination of 4.0 µmol/L 2-PCPA and 2 µmol/L T1A02-135; P4_T3: combination of 4.0 µmol/L 2-PCPA and 3 µmol/L T1A02-135; P5_T0: 5.0 µmol/L 2-PCPA; P5_T1: combination of 5.0 µmol/L 2-PCPA and 1.0 µmol/L T1A02-135; P5_T2: combination of 5.0 µmol/L 2-PCPA and 2 µmol/L T1A02-135; and P5_T3: combination of 5.0 µmol/L 2-PCPA and 3 µmol/L T1A02-135.

### <Example 28>

Figure 28 shows the results of analyzing human umbilical cord blood CD34⁺ cells cultured with compound(s) in a Soluplus-based medium for 10 days using Flow Cytometry. The vertical axis shows the number of CD41⁺CD34⁺ cells. The horizontal axis shows the compounds and their contents, which are the same as in Figure 27.

### <Example 29>

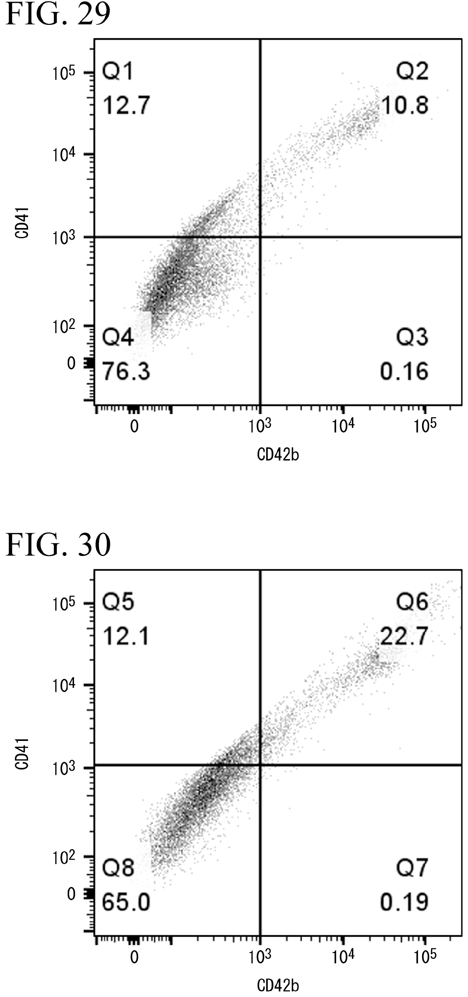

Figure 29 shows the ratio of mature megakaryocytes (CD41⁺CD42b⁺ cells) to the cell population obtained after culturing human umbilical cord blood CD34⁺ cells with compound(s) in a Soluplus-based medium for 14 days (expansion rate 27.0-fold, CD34 positive rate 73.4%, viable cell rate 88.1%, CD41 positive rate 15.2%), followed by 7 days of culture using a megakaryocyte differentiation medium (StemSpan SFEM from StemCell Technologies, catalog number ST-09600).

### <Example 30>

Figure 30 shows the ratio of mature megakaryocytes (CD41⁺CD42b⁺ cells) to the cell population obtained after culturing human umbilical cord blood CD34⁺ cells with compound(s) in a Soluplus-based medium for 14 days (expansion rate 27.0-fold, CD34 positive rate 73.4%, viable cell rate 88.1%, CD41 positive rate 15.2%), followed by 14 days of culture using a megakaryocyte differentiation medium (StemSpan SFEM from StemCell Technologies, catalog number ST-09600).

From the above results, it was found that according to the present disclosure, a composition and a medium for cell culture can be provided.

### INDUSTRIAL APPLICABILITY

According to the present disclosure, a composition and a medium for cell culture can be provided.

Furthermore, according to the present disclosure, a cell population that can be transplanted into patients with diseases such as cancer and immunodeficiency, and a method for producing the same can be provided.

Furthermore, according to the present disclosure, a therapeutic agent comprising said cell population can be provided.

## Claims

1. A composition (1) comprising component (A), which is a KDM5 degrader, and component (B), which is an LSD1 inhibitor, or
a composition (2) comprising component (A), which is a KDM5 degrader, wherein
in composition (2), said component (A) comprises at least one component selected from the group consisting of a compound represented by the following general formula (I) and a salt thereof, and a compound represented by the following general formula (II) and a salt thereof: 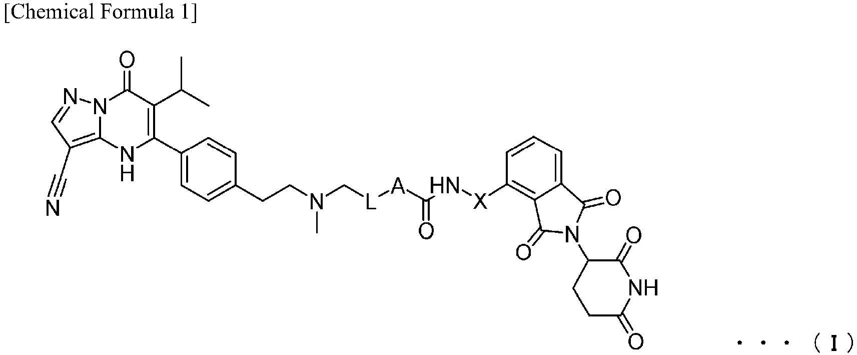
in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10; 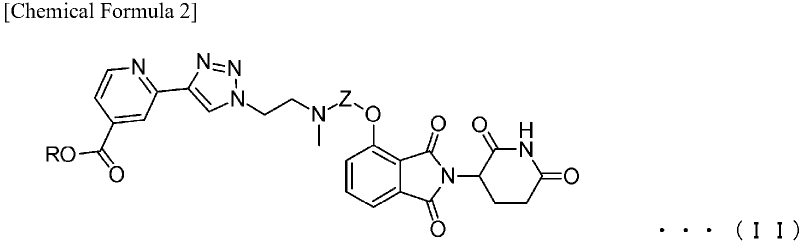
in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

2. The composition according to claim 1, wherein said composition is composition (1), and
said component (A) comprises at least one component selected from the group consisting of a compound represented by the following formula (I) and a salt thereof, and a compound represented by the following general formula (II) and a salt thereof: 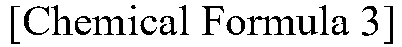 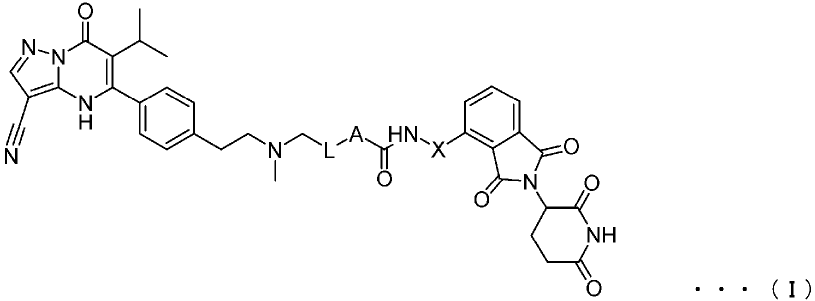
in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10; 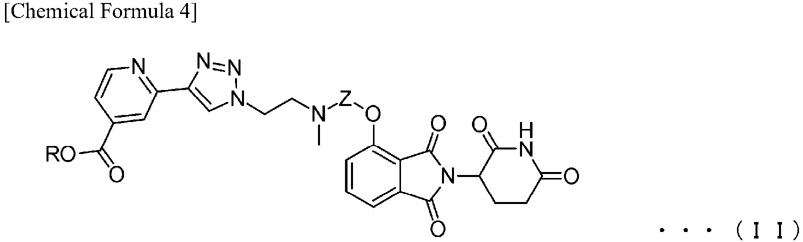
in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

3. The composition according to claim 1, wherein
in said general formula (I), A is a single bond or phenylene group, L is a linear alkylene group having 3 to 10 carbon atoms, X is a group represented by -(CH₂CH₂O)□-, -(CH₂CH₂CH₂O)□-, -(CH(CH₃)CH₂O)□-, or -(CH₂)ₜO-, s is a number from 1 to 5, and t is a number from 2 to 10, and
in said general formula (II), R is a hydrogen atom, or a linear alkyl group having 1 to 6 carbon atoms, and Z is a substituted or unsubstituted linear alkylene group having 2 to 18 carbon atoms, or a substituted or unsubstituted branched alkylene group having 2 to 18 carbon atoms.

4. The composition according to claim 1, wherein
in said general formula (I), A is a single bond, p-phenylene group, or m-phenylene group, L is a linear alkylene group having 3 to 10 carbon atoms, X is a group represented by -(CH₂CH₂O)□-, -(CH₂CH₂CH₂O)□-, or - (CH₂)ₜO-, s is a number from 1 to 5, and t is a number from 2 to 10, and
in said general formula (II), R is a linear alkyl group having 1 to 6 carbon atoms, and Z is a substituted or unsubstituted linear alkylene group having 3 to 16 carbon atoms, or a substituted or unsubstituted branched alkylene group having 3 to 16 carbon atoms.

5. The composition according to claim 1, wherein said component (A) is at least one selected from the group consisting of a compound represented by the following general formula (I-1) and a salt thereof, a compound represented by the following general formula (I-2) and a salt thereof, a compound represented by the following general formula (I-3) and a salt thereof, and a compound represented by the following general formula (II-1) and a salt thereof: 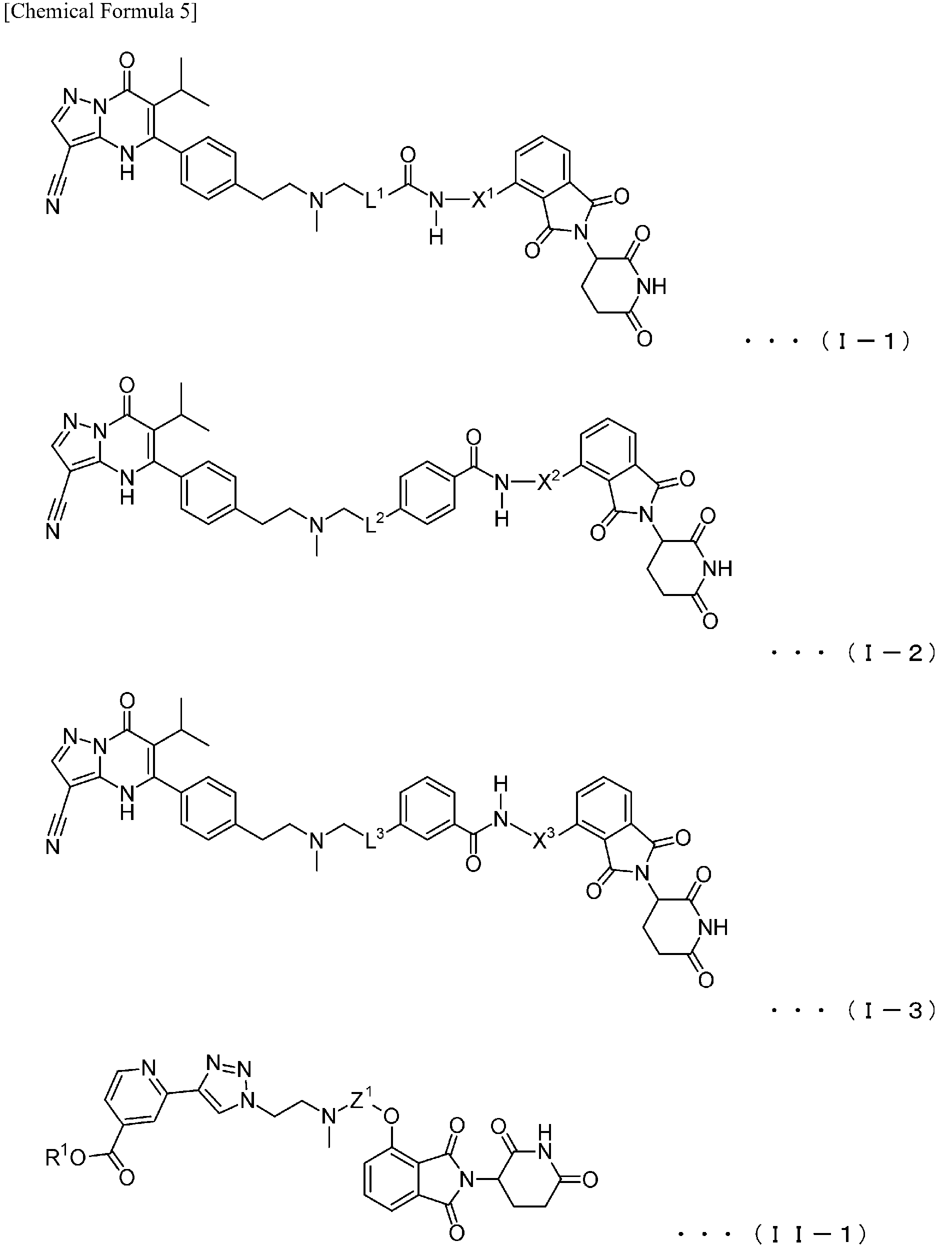
in general formula (I-1), L¹ is a linear alkylene group having 3 to 10 carbon atoms, X¹ is a group represented by -(CH₂CH₂O)□-, and s is a number from 1 to 5,
in formula (I-2), L² is a linear alkylene group having 3 to 10 carbon atoms, X² is a group represented by - (CH₂CH₂O)□- or -(CH₂)ₜO-, s is a number from 1 to 5, and t is a number from 2 to 10,
in formula (I-3), L³ is a linear alkylene group having 3 to 10 carbon atoms, X³ is a group represented by - (CH₂CH₂O)□- or -(CH₂)ₜO-, s is a number from 1 to 5, and t is a number from 2 to 10, and
in general formula (II-1), R¹ is a linear alkyl group having 1 to 3 carbon atoms, and Z¹ is a substituted or unsubstituted linear alkylene group having 3 to 14 carbon atoms, or a substituted or unsubstituted branched alkylene group having 3 to 14 carbon atoms.

6. The composition according to claim 5, wherein said component (A) is:
a compound of said general formula (I-1), wherein L¹ is a linear alkylene group having 5 carbon atoms, and X¹ is a group represented by -(CH₂CH₂O)□-, and s is 3;
a compound of said general formula (I-1), wherein L¹ is a linear alkylene group having 8 carbon atoms, and X¹ is a group represented by -(CH₂CH₂O)□-, and s is 3;
a compound of said general formula (I-2), wherein L² is a linear alkylene group having 5 carbon atoms, and X² is a group represented by -(CH₂CH₂O)□-, and s is 3;
a compound of said general formula (I-2), wherein L² is a linear alkylene group having 5 carbon atoms, and X² is a group represented by -(CH₂)ₜO-, and t is 6;
a compound of said general formula (I-3), wherein L³ is a linear alkylene group having 5 carbon atoms, and X³ is a group represented by -(CH₂CH₂O)□-, and s is 3;
a compound of said general formula (I-3), wherein L³ is a linear alkylene group having 5 carbon atoms, and X³ is a group represented by -(CH₂)ₜO-, and t is 6; or
a compound of said general formula (II-1), wherein R¹ is a methyl group, and Z¹ is an unsubstituted linear alkylene group having 9 carbon atoms.

7. The composition according to claim 1, wherein said composition is composition (1), and
said component (B) comprises at least one component selected from the group consisting of tranylcypromine and a salt thereof.

8. The composition according to claim 6, wherein said composition is composition (1), and
said component (B) comprises at least one component selected from the group consisting of tranylcypromine and a salt thereof.

9. A medium comprising the composition according to any one of claims 1 to 8.

10. The medium according to claim 9, wherein said medium is a medium for culturing human hematopoietic stem cells.

11. A cell population obtained by culturing in the medium according to claim 9.

12. The cell population according to claim 11, wherein said cell population comprises human hematopoietic stem cells.

13. At least one therapeutic agent selected from the group consisting of a cancer therapeutic agent, an immunodeficiency therapeutic agent, and a multiple myeloma therapeutic agent, comprising cells obtained by culturing in the medium according to claim 9.

14. A method for producing cells, comprising a step of culturing cells using the medium according to claim 9.

15. The method for producing cells according to claim 14, wherein said culturing step comprises obtaining a medium from said cells and said composition.

16. The method for producing cells according to claim 14, comprising a step of culturing the cells cultured in said culturing step using another medium.

17. The method for producing cells according to claim 14, wherein said cultured cells are a cell population comprising human hematopoietic stem cells.

18. A cell population produced by the method according to claim 14.

19. The cell population according to claim 18, wherein said cell population comprises human hematopoietic stem cells.

20. At least one therapeutic agent selected from the group consisting of a cancer therapeutic agent, an immunodeficiency therapeutic agent, and a multiple myeloma therapeutic agent, comprising cells obtained using the method according to claim 14.

21. Use of an agent comprising at least one component selected from the group consisting of a compound represented by the following general formula (I) and a salt thereof, and a compound represented by the following general formula (II) and a salt thereof, for manufacture of a medium for expansion of hematopoietic stem cells: 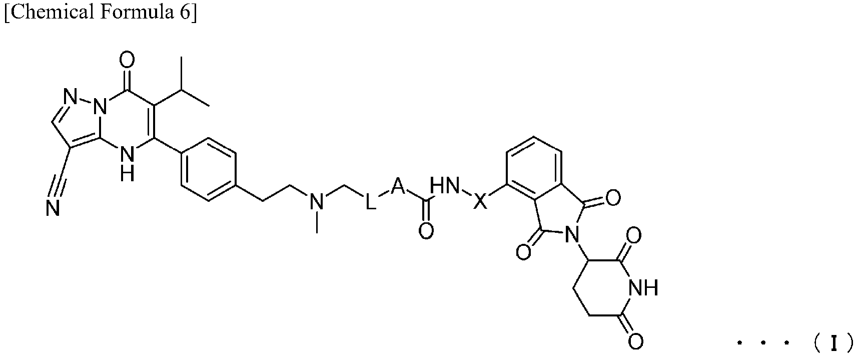 in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)□-, - (CH₂CH₂CH₂O)□-, -(CH(CH₃)CH₂O)□-, or -(CH₂)ₜO-, s is a number from 1 to 8, and t is a number from 2 to 10; 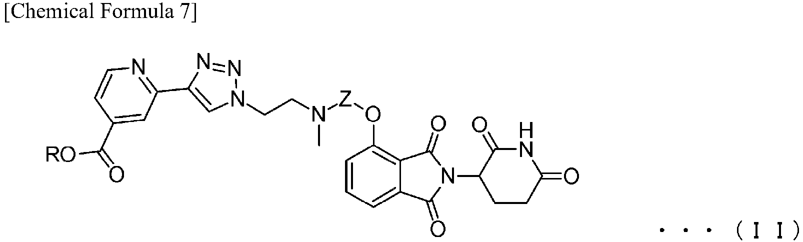 in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

22. Use of an agent comprising at least one component selected from the group consisting of a compound represented by the following general formula (I) and a salt thereof, and a compound represented by the following general formula (II) and a salt thereof, as an agent for a medium for expansion of hematopoietic stem cells: 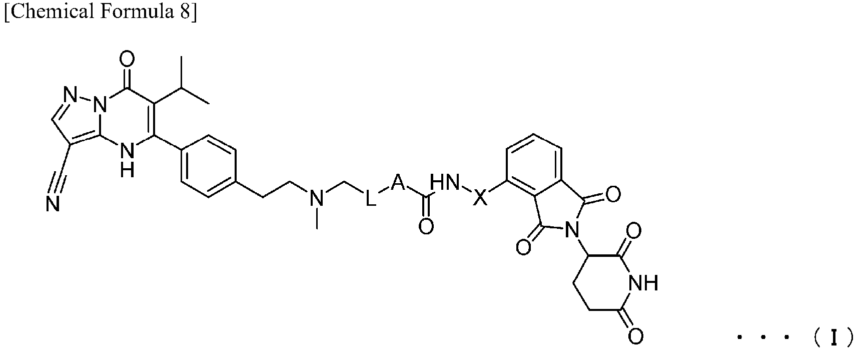 in general formula (I), A is a single bond, or a substituted or unsubstituted divalent aromatic hydrocarbon group, L is a substituted or unsubstituted linear alkylene group having 1 to 13 carbon atoms, or a substituted or unsubstituted branched alkylene group having 1 to 13 carbon atoms, X is a group represented by -(CH₂CH₂O)ₛ-, - (CH₂CH₂CH₂O)ₛ-, -(CH(CH₃)CH₂O)ₛ-, or -(CH₂)ₜO-, s is a number from 1 to 8, and t is a number from 2 to 10; 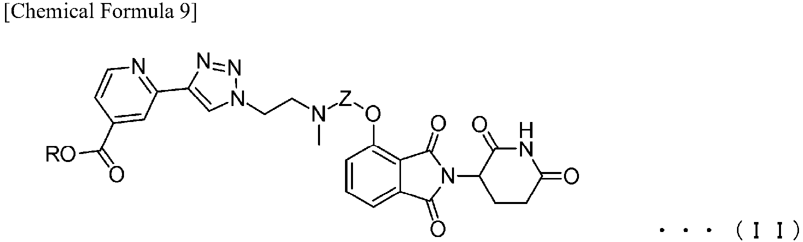 in general formula (II), R is a hydrogen atom, a substituted or unsubstituted linear alkyl group having 1 to 6 carbon atoms, or a substituted or unsubstituted branched alkyl group having 1 to 6 carbon atoms, and Z is a substituted or unsubstituted linear alkylene group having 2 to 20 carbon atoms, or a substituted or unsubstituted branched alkylene group having 2 to 20 carbon atoms.

23. Use of the composition according to any one of claims 1 to 8 for expansion of hematopoietic stem cells.

24. The use according to claim 23, wherein said hematopoietic stem cells are intended for differentiation into megakaryocytes.
